# EUROPEAN PATENT SPECIFICATION

(11) **EP 4 534 690 B1**
(45) Date of publication and mention of the grant of the patent: **14.10.2026**
(21) Application number: 25156820.0
(22) Date of filing: 01.02.2021
(51) Int. Cl.: C12N 15/11, C40B 40/06, C12Q 1/6806, C12Q 1/6853, C12Q 1/6874, C12Q 1/6832

(54) **SYSTEMS AND METHODS FOR TARGETED NUCLEIC ACID CAPTURE**
SYSTEME UND VERFAHREN ZUM GEZIELTEN NUKLEINSÄUREEINFANG
SYSTÈMES ET PROCÉDÉS DE CAPTURE CIBLÉE D'ACIDES NUCLÉIQUES

(30) Priority: 31.01.2020 US 202062968847 P; 09.03.2020 US 202062987232 P; 12.03.2020 US 202062988859 P
(43) Date of publication of application: 09.04.2025
(62) Document number(s) of the earlier application(s) in accordance with Art. 76 EPC: 21747390.9 / 4 097 231
(73) Proprietor: Agilent Technologies, Inc., Santa Clara, CA 95051 (US)
(72) Inventor: LIN, Shengrong, Fremont, CA 94555 (US); BAO, Yun, Fremont, CA 94539 (US); WANG, Heng, Pleasanton, CA 94566 (US); ZHAO, Grace, Palo Alto, CA 94306 (US)
(74) Representative: Grünecker Patent- und Rechtsanwälte PartG mbB

(56) References cited:
- WO-A1-2010/030683
- WO-A1-2010/051978
- WO-A1-2010/091870
- WO-A1-2014/015098
- MICHAEL W SCHMITT ET AL: "Sequencing small genomic targets with high efficiency and extreme accuracy", NATURE METHODS, vol. 12, no. 5, 1 May 2015 (2015-05-01), New York, pages 423 - 425, XP055623852, ISSN: 1548-7091, DOI: 10.1038/nmeth.3351

## Description

### CROSS-REFERENCE

This application claims the benefit of U.S. Provisional Application No. 62/968,847, filed January 31, 2020, U.S. Provisional Application No. 62/987,232, filed March 9, 2020, and U.S. Provisional Application No. 62/988,859, filed March 12, 2020.

This application is related to the following co-pending patent application: International Application No. PCT/US2019/062508, filed on November 20, 2019.

### BACKGROUND

Nucleic acid target capture methods can allow specific genes, exons, and other genomic regions of interest to be enriched, e.g., for targeted sequencing. However, target capture-based sequencing methods can involve cumbersome lengthy protocols and costly processes, as well as a low on-target rate for a small capture panel (e.g., less than 500 probes). Moreover, current methods for nucleic acid target capture can be ill-suited for low input and damaged DNA because of a low recovery rate.

Bisulfite conversion can be a useful technique to study the methylation pattern of nucleic acid molecules. However, bisulfite conversion can damage nucleic acids by creating truncations for example. If a next-generation sequencing (NGS) DNA library is treated with bisulfite, a substantial amount of the nucleic acids can be damaged and be unable to be recovered in the subsequent amplification steps, and thereby provide a low recovery rate. Moreover, because the bisulfite conversion can result in single stranded or fragmented DNA and reduced sequence complexity, converted DNA can be a difficult input for conventional adaptor-ligation based library construction. Bisulfite treated cell-free (cfDNA) or circulating tumor cell DNA (ctDNA) with typically small initial input can present a bigger challenge given the low recovery rate (e.g. 5% or less for bisulfite treated cfDNA). A methylation-sensitive enzymatic treatment can also be performed to convert the methylated cytosine. However, the enzyme-based approach can still suffer from the loss of methylation status during the long and multi-step process, leading to a low recovery rate.

Methylation analysis in cell-free DNA holds great potential for early cancer detection. In the plasma of early stage cancer patient, the tumor content is estimated to be less than 0.1%, often down to 0.01% or lower, and therefore requires a highly sensitive assay. Currently there are two major approaches used for cancer screening: the global approach, including whole genome bisulfite sequencing (WGBS), reduced representation bisulfite sequencing (RRBS) or affinity-based enrichment, and large targeted panels containing 10,000 or more of potential methylation markers. Targeted Methylation Sequencing (TMS) provides the most sensitive and specific analysis of methylation markers. However, the sensitivity and specificity of conventional TMS is compromised by low efficiency and low recovery of target enrichment, and further hampered by background noise associated with large panels. There is a need for methods for in-depth analysis using a small, focused, cancer-specific methylation biomarker panel.

Therefore, there is a need for a more efficient, easy to use, fast, flexible, and practical target nucleic acid capture methods and improved methods for analyzing bisulfite treated nucleic acid especially for the low-input samples such as cfDNA. The method disclosed herein can be used for pre-amplification and pre-bisulfite conversion hybridization-based capture for very low DNA input samples.

WO 2010/030683 A1 relates to methods for the indirect capture of a target gene by using a single bi-functional capture probe that hybridizes to a target nucleic acid sequence and to a capture probe. Schmitt et al. (Nature Methods, vol. 12(5): 423-425, 2015) relates to methods for preparing a sequencing library based on sequential rounds of target enrichment to increase the on-target depth.

### SUMMARY

The present invention is defined in the claims. Disclosed herein is a method comprising: obtaining a template nucleic acid molecule comprising an adaptor at a 5' end or a 3' end of the template nucleic acid molecule; hybridizing a first target specific region of a first bridge probe to a first target sequence of the template nucleic acid molecule, wherein a first adaptor landing sequence of the first bridge probe is bound to a first bridge binding sequence of an adaptor anchor probe; and hybridizing a second target specific region of a second bridge probe to a second target sequence of the template nucleic acid molecule, wherein a second adaptor landing sequence of the second bridge probe is bound to a second bridge binding sequence of the adaptor anchor probe. The method can further comprise attaching the adaptor to the 5' end or the 3' end of a sample nucleic acid molecule, thereby generating a template nucleic acid molecule comprising the adaptor. The method can further comprise attaching the adaptor to the 5' end or the 3' end of a sample nucleic acid molecule, and attaching an adaptor to the 3' end or 5' end respectively of the template nucleic acid molecule comprising the adaptor, thereby generating a template nucleic acid molecule comprising an adaptor on each end. The method can further comprise hybridizing an adaptor primer to the adaptor attached to the 3' end of the template nucleic acid molecule hybridized to the first bridge probe and the second bridge probe; and extending a 3' end of the adaptor primer, thereby generating an extension product. The method can further comprise sequencing the extension product.

The first adaptor landing sequence of the first bridge probe can be bound to the first bridge binding sequence of the adaptor anchor probe before the hybridizing to the first target specific region. The first adaptor landing sequence of the first bridge probe can be bound to the first bridge binding sequence of the adaptor anchor probe after the hybridizing to the first target specific region. The second adaptor landing sequence of the second bridge probe can be bound to the second bridge binding sequence of the adaptor anchor probe before the hybridizing to the second target specific region. The second adaptor landing sequence of the second bridge probe can be bound to the second bridge binding sequence of the adaptor anchor probe after the hybridizing to the second target specific region.

The method can further comprise hybridizing the first landing sequence of the first bridge probe to the first bridge binding sequence of the adaptor anchor probe. The method can further comprise hybridizing the second landing sequence of the second bridge probe to the second bridge binding sequence of the adaptor anchor probe. The adaptor anchor probe can further comprise a spacer located between the first bridge binding sequence and the second bridge binding sequence. The adaptor can comprise molecular barcodes.

The adaptor anchor probe can comprise a binding moiety. The binding moiety can be attached to a support. The support can be a bead. The bead can be a streptavidin bead. The binding moiety can be a biotin.

The first bridge probe can comprise a binding moiety. The binding moiety can be attached to a support. The support can be a bead. The bead can be a streptavidin bead. The binding moiety can be a biotin.

The template nucleic acid molecule can comprise single-stranded DNA. The template nucleic acid molecule can comprise cell-free nucleic acid from a biological sample. The cell-free nucleic acid can comprise cell-free DNA. The cell-free DNA can comprise circulating tumor DNA. The template nucleic acid molecule can comprise damaged DNA.

The method can further comprise treating the template nucleic acid molecule with a methylation assay reagent, after the hybridizing of the first target specific region and the hybridizing of the second target specific region. The methylation assay reagent can be bisulfide, or an enzyme which modifies methylated cytosines. The method can further comprise hybridizing a third target specific region of a third bridge probe to a third target sequence of a template nucleic acid molecule, wherein a third adaptor landing sequence of the third bridge probe is bound to a third bridge binding sequence of an adaptor anchor probe. The method can further comprise hybridizing a fourth target specific region of a fourth bridge probe to a fourth target sequence of a template nucleic acid molecule, wherein a fourth adaptor landing sequence of the fourth bridge probe is bound to a fourth bridge binding sequence of an adaptor anchor probe

The method can further comprise attaching an adaptor to a 5' end or a 3' end of the template nucleic acid molecule prior to the hybridizing the first bridge probe and the hybridizing the second bridge probe. The method can further comprise hybridizing an adaptor primer to the adaptor attached to the 3' end of the template nucleic acid molecule hybridized to the first bridge probe and the second bridge probe; and extending a 3' end of the adaptor primer, thereby generating an extension product. The method can further comprise sequencing the extension product.

The hybridizing of the adaptor primer can be performed prior to treatment with the bisulfite. The hybridizing of the adaptor primer can be performed after treatment with the bisulfite. The adaptor primer can be designed based on the adaptor after treatment with the bisulfite, wherein non-methylated cytosine in the adaptor is converted to uracil during the treatment. The first adaptor landing sequence of the first bridge probe can be bound to the first bridge binding sequence of the adaptor anchor probe before the hybridizing to the first target specific region. The first adaptor landing sequence of the first bridge probe can be bound to the first bridge binding sequence of the adaptor anchor probe after the hybridizing to the first target specific region. The second adaptor landing sequence of the second bridge probe can be bound to the second bridge binding sequence of the adaptor anchor probe before the hybridizing to the second target specific region. The second adaptor landing sequence of the second bridge probe can be bound to the second bridge binding sequence of the adaptor anchor probe after the hybridizing to the second target specific region.

The method can further comprise hybridizing the first landing sequence of the first bridge probe to the first bridge binding sequence of the adaptor anchor probe. The method can further comprise hybridizing the second landing sequence of the second bridge probe to the second bridge binding sequence of the adaptor anchor probe. The adaptor anchor probe can further comprise a spacer located between the first bridge binding sequence and the second bridge binding sequence. The adaptor can comprise molecular barcodes.

The adaptor anchor probe can comprise a binding moiety. The binding moiety can be attached to a support. The support can be a bead. The bead can be a streptavidin bead. The binding moiety can be a biotin. The first bridge probe can comprise a binding moiety. The binding moiety can be attached to a support. The support can be a bead. The bead can be a streptavidin bead. The binding moiety can be a biotin. The template nucleic acid molecule can comprise single-stranded DNA. The template nucleic acid molecule can comprise cell-free nucleic acid from a biological sample. The cell-free nucleic acid can comprise cell-free DNA. The cell-free DNA can comprise circulating tumor DNA. The template nucleic acid molecule can comprise damaged DNA.

Disclosed herein is a nucleic acid complex comprising: a template nucleic molecule, wherein a 5' end or a 3' end of the template nucleic molecule is attached to an adaptor, wherein a first target sequence of the template nucleic acid molecule is hybridized to a first target specific region of a first bridge probe and a second target sequence of the template nucleic acid molecule is hybridized to a second target specific region of a second bridge probe, and wherein a first adaptor landing sequence of the first bridge probe is bound to a first bridge binding sequence of an adaptor anchor probe and a second adaptor landing sequence of the second bridge probe is bound to a second bridge binding sequence of the adaptor anchor probe.

Disclosed herein is a method of sequential enrichment comprising obtaining a sample comprising a plurality of nucleic acid molecules; performing a first target enrichment to enrich for nucleic acid molecules comprising sequences corresponding to a first panel of one or more genome regions, thereby generating a first enriched sample comprising nucleic acids enriched for sequences corresponding to the first panel of one or more genome regions and a remaining sample comprising nucleic acids depleted for sequences corresponding to the first panel of one or more genome regions; and performing a second target enrichment upon the remaining sample to enrich for nucleic acid molecules comprising sequences corresponding to a second panel of one or more genome regions, thereby generating a second enriched sample comprising nucleic acids enriched for sequences corresponding to the second panel of one or more genome regions; wherein the first panel of one or more genome regions and the second panel of one or more genome regions are different.

The method further comprises performing a first analysis of the first enriched sample and a second analysis of the second enriched sample.

The first analysis can be a sequence analysis, and the second analysis can be a methylation analysis.

In some cases, the first analysis is a first sequence analysis, and the second analysis is a second sequence analysis, wherein the first sequence analysis is performed at a different depth of sequencing than the second sequence analysis.

In some cases, the sample is a cfDNA sample.

Target enrichment for a genome region of the panel of one or more genome regions comprises a target enrichment by hybridization.

Target enrichment for a genome region of the panel of one or more genome regions comprises: hybridizing a first target specific region of a first bridge probe to a first target sequence of a molecule with a sequence corresponding to the genome region, wherein a first adaptor landing sequence of the first bridge probe is bound to a first bridge binding sequence of an adaptor anchor probe; and hybridizing a second target specific region of a second bridge probe to a second target sequence of the molecule with a sequence corresponding to the genome region, wherein a second adaptor landing sequence of the second bridge probe is bound to a second bridge binding sequence of the adaptor anchor probe.

**In** some cases, the adaptor anchor probe comprises a binding moiety.

The method can further comprise attaching the binding moiety to a support and separating the support with attached binding moiety from the unbound nucleic acids.

**In** some cases, or second panel of genomic regions comprises promoter regions.

**In** some cases, the first or second panel of genomic regions comprises intronic regions.

**In** some cases, the first or second panel of genomic regions comprises exonic regions.

**In** some cases, the method further comprises attaching adaptors to the 5' end or the 3' ends of nucleic acid molecules of the plurality of nucleic acid molecules, thereby generating a library of nucleic acid molecules comprising adaptors.

**In** some cases, the second enriched sample is bisulfite treated and subjected to a sequencing reaction.

**In** some cases, the number of informative reads of the sequencing reaction is at least 60%, 65%, 70%, 75%, 80%, 85% , 90% or 95% of the number of informative reads that could be obtained from the sample if it was subjected to a single target enrichment to enrich for nucleic acid molecules comprising sequences corresponding to a second panel of one or more genome regions.

**In** some cases, the method further comprises performing a third target enrichment upon and a second remaining sample, comprising nucleic acids depleted for sequences corresponding to the first panel and second panel of one or more genome regions, to enrich for nucleic acid molecules comprising sequences corresponding to a third panel of one or more genome regions, thereby generating a third enriched sample comprising nucleic acids enriched for sequences corresponding to the third panel of one or more genome regions; wherein the first panel of one or more genome regions, the second panel of one or more genome regions, and the third panel of one or more genome regions are different.

In some cases, the method further comprises hybridizing a third target specific region of a third bridge probe to a third target sequence of the molecule with a sequence corresponding to the genome region, wherein a third adaptor landing sequence of the third bridge probe is bound to a third bridge binding sequence of the adaptor anchor probe.

In some cases, the method further comprises hybridizing a fourth target specific region of a fourth bridge probe to a fourth target sequence of the molecule with a sequence corresponding to the genome region, wherein a fourth adaptor landing sequence of the fourth bridge probe is bound to a fourth bridge binding sequence of the adaptor anchor probe.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** illustrates one embodiment of a synergistic, indirect hybridization capture of a template nucleic acid molecule. In this embodiment, a library of the template nucleic acid molecules is constructed prior to the indirect hybridization.
**FIGS. 2A-2B** illustrate one embodiment of a synergistic, indirect hybridization capture of a template nucleic acid molecule for methylation sequencing. **FIG. 2A** shows a synergistic, indirect hybridization capture of the template nucleic acid molecule and **FIG. 2B** shows subsequent bisulfite conversion of the captured templated nucleic acid molecule.
**FIG. 3** shows a workflow for synergistic, indirect hybridization capture and targeted methylation sequencing (SICON-TMS) of a template nucleic acid molecule.
**FIG. 4** shows a schematic view of a synergistic, indirect hybridization.
**FIGS. 5A-5D** show schematic views of different hybridization systems. **FIG. 5A** illustrates a non-synergistic, direct hybridization. **FIG. 5B** illustrates a synergistic, direct hybridization. **FIG. 5C** illustrates a synergistic, indirect hybridization. **FIG. 5D** illustrates a non-synergistic, indirect hybridization.
**FIGS. 6A-6B** illustrate schematic views of synergistic, indirect hybridizations using adaptor anchor probes with or without spacers in-between the bridge binding sequences of adaptor anchor probes. **FIG. 6A** shows a schematic view of the synergistic, indirect hybridization with adaptor anchor probe comprising the spacers. **FIG. 6B** shows the synergistic, indirect hybridization with adaptor anchor probe lacking the spacers.
**FIG. 7** shows a sequencing coverage of a 15-target panel using synergistic, indirect capture method.
**FIGS. 8A-8B** shows sequencing coverages of a panel of 76 human gene targets (human ID) using two different hybridization methods. **FIG. 8A** shows the coverage by a pre-amplification capture by synergistic, indirect hybridization. **FIG. 8B** shows the coverage by a post-amplification capture by direct hybridization.
**FIG. 9** shows a result of a targeted methylation sequencing assay after synergistic, indirect capture of cfDNA extracted from non-cancerous individual.
**FIG. 10** illustrates a result of a targeted methylation sequencing assay showing a linear relationship between the expected amount of spike-in methylated DNA and the measured value.
**FIGS. 11A and 11B** show the molecule methylation scatter pattern of DMR1 in normal colon tissue and colon cancer tissue genomic DNA respectively.
**FIGS. 12A and 12B** show the molecule methylation scatter pattern of DMR2 in normal colon tissue and colon cancer tissue genomic DNA respectively.
**FIGS. 13A and 13B** show the molecule methylation scatter pattern of DMR1 and DMR2 in a health individual's plasma cfDNA and a colon cancer patient's plasma cfDNA respectively.
**FIG. 14** illustrates a schematic for sequential target enrichment from a sample.
**FIG. 15** illustrates mutations identified in CRC cfDNA samples in Example 11.
**FIG. 16** illustrates methylation scores from the stand alone and dual analysis TMS.
**FIG. 17** illustrates the informative molecule counts from stand alone and dual analysis TMS.
**FIG. 18** illustrates sensitivity of variant allele detection in a personalized panel analysis.

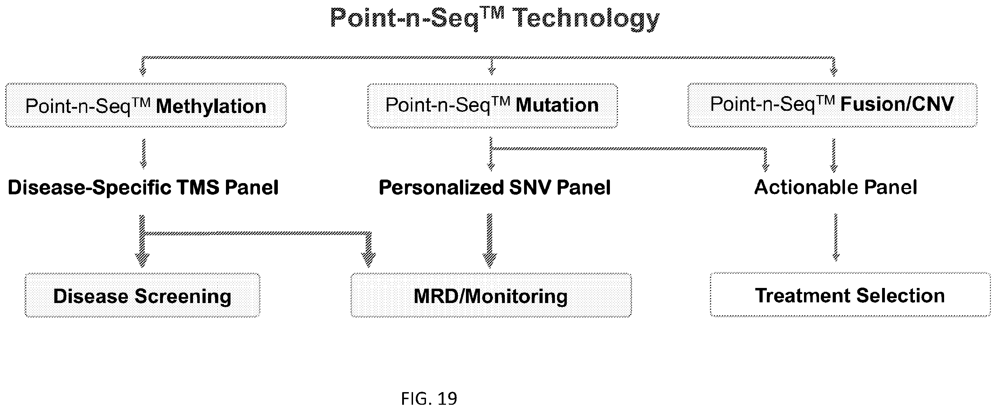

**FIG. 19** illustrates implementations of the Point-n-Seq^{™} technology.

### DETAILED DESCRIPTION

CfDNA based liquid biopsy using methylation and mutation analysis can be used for cancer early detection and management. Provided herein are systems and methods for combined analyses from limited quantities of nucleic acid samples. For example, provided herein are systems and methods for combined Targeted Methylation Sequencing (TMS) and mutation analysis from a limited DNA sample. These systems and methods may be of particular use for cfDNA samples, which can be low in quantity.

Broad but tissue-specific methylation changes in cancer genomes can be used for sensitive detection of circulating tumor (ctDNA) in plasma from early stage or recurrent cancer patients. However, the sensitivity of methylation analyses may be compromised by low efficiency in recovering methylation markers in the process, and the specificity is sometimes further hampered by the approach of including noisy non-specific markers to compensate for the low detection sensitivity. Moreover, while methylation analysis can hold advantages for early cancer detection, the actionable mutation can directly provide information to guide treatment selection and further increase assay specificity. The yield of cfDNA from limited clinical blood samples can be of low quantity, which can be a major challenge for performing multiple analyses from one sample, thus an assay that can detect both methylation and mutation can provide improvements for clinical research and diagnostic assays.

This disclosure provides an improved technology designed for targeted methylation and mutation combined analysis in cfDNA: Point-n-Seq, featuring an enrichment of target molecules directly from cfDNA, before cytosine conversion and amplification. This technology can enable small focused panels that interrogate the methylation or mutation status of at least 10, 100, 1000 or more than 1000 markers. Provided herein is a colorectal cancer (CRC) panel designed covering 100 methylation markers and >350 hotspot mutations from 22 genes. Point-n-Seq TMS can be used for small focused methylation and mutation combined panel sequencing using cfDNA. Point-n-Seq TMS can be used in the development of practical and cost-effective methylation assays for research and clinical use.

Utilizing an ultra-efficient pre-conversion/pre-amplification capture Point-n-Seq can be used for disease-focused methylation and mutation panel enrichment. Point-n-Seq TMS enables analysis of small focused methylation and mutation panels using cfDNA. Point-n-Seq TMS can be used in practical and cost-effective methylation assays for research and clinical use.

Also provided herein are systems and methods for synergistic indirect capture of nucleic acid for sequencing (SICON-SEQ, also termed Point-n-SEQ). The systems and methods disclosed herein allow efficient capture and enrichment of nucleic acid materials. SICON-SEQ/Point-n-SEQ can be performed for capture enrichment after library construction by attachment of adaptors to template nucleic acid materials. In some embodiments, SICON-SEQ can be performed before library construction. SICON -SEQ can be performed without the library construction by adaptor attachment. SICON-SEQ methods disclosed herein can allow a short turn-around time and simple workflow. SICON-SEQ can be used to handle low input samples such cell-free DNA (cfDNA), therefore can be suitable for methylation sequencing analysis.

Disclosed herein are methods comprising indirect hybridization of the template nucleic acid molecule with an adaptor anchor probe through hybridization of two or more bridge probes to the template nucleic acid. The two or more bridge probes can be designed to hybridize to particular target sequences in the template nucleic acid molecule and thereby can be hybridized to the target template. An adaptor anchor probe in turn can be designed to hybridize to the bridge probes, thereby creating an assembly of hybridized nucleic acid molecules. The multi-structure hybridization assembly can act synergistic to provide more stability to the assembly. The hybridized template nucleic acid molecule can be subsequently treated with bisulfite for methylation sequencing.

### I. Indirect Capture by Hybridization

The target probe hybridization can be facilitated by synergistic interaction of template nucleic acid and two or more probes that form a hybridization assembly. The multi-complex assembly can stabilize the hybridization interaction between the template and the target probes such as bridge probes. A bridge probe can comprise a target specific region that hybridizes to a target region of the template and adaptor landing sequence (ALS) that hybridizes to bridge binding sequence (BBS) of an adaptor anchor probe. The hybridizations between the template and the bridge probe and between the bridge probe and the adaptor anchor probe can form multi-complex assembly.

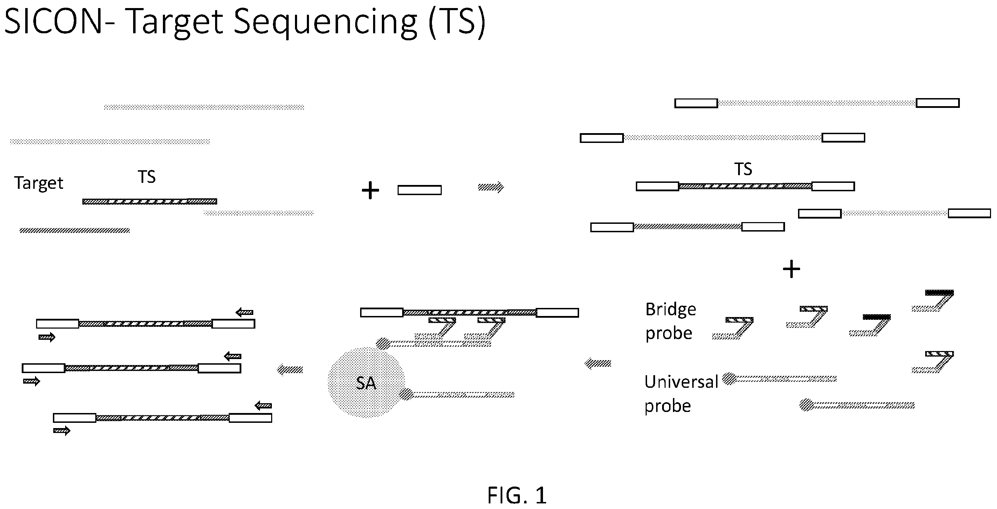

More than two bridge probes pre target region can be used in the methods disclosed herein. For example, at least 2, 3,4, 5, 6, 7, 8, 9, 10, 25, 50, 75, 100, or more bridge probes can be used to bridge the template and the adaptor anchor probe. The synergistic indirect capture of nucleic acid for sequencing (SICON-SEQ) methods can further comprise hybridizing a second target specific region of a second bridge probe to a second target sequence of the template nucleic acid molecule, wherein a second adaptor landing sequence of the second bridge probe can be bound to a second bridge binding sequence of the adaptor anchor probe (FIG. 1). In some cases, the SICON-SEQ can be conducted after attachment of adaptors to the template nucleic acid molecules to generate a library (FIG. 1). The library can be next generation sequencing (NGS) library.

The bridge probes can further comprise linkers that connect the target specific region and the adaptor landing sequence. The adaptor anchor can comprise one or more spacers in between the bridge binding sequences. The presence of the one or more spacers can improve the efficiency of the hybridization capture and increase the specificity of the capture.

The template nucleic acid can be captured and enriched from low-input samples such as cell-free DNA (cfDNA) and circulating tumor DNA (ctDNA). The capture and enrichment can be done by the indirect association with adaptor anchor probe through hybridization with bridge probe. The bridge probe and/or adaptor anchor probe can comprise one or more binding moieties. The binding moiety can be a biotin. The binding moieties can be attached to a support. The support can be a bead. The bead can be a streptavidin bead.

### II. Workflows for Methylation Analysis

Provided herein are methods for methylation analysis of nucleic acids. The methylation analysis can be done by bisulfite treatment. The bisulfite treated nucleic acids can be used to study methylation of the nucleic acids. The bisulfite treatment can convert unmethylated cytosines to uracils. Methylation of a cytosine (e.g., 5'-methylctyosine) can prevent bisulfite from converting methylated cytosine to uracil.

The template nucleic acid molecules can be treated with bisulfite either before or after hybridization capture using capture probe or bridge probe/adaptor anchor probe. In some cases, the hybridized template nucleic acid molecules can be treated with bisulfite. Formation of double strand sequence (e.g., between a TS of template and TSR of a capture probe) can protect against conversion of cytosines in the hybridized region to uracils during bisulfite treatment. The double stranded sequence formed by the hybridization of the capture probe to the template or the bridge probe to the template and to an adaptor anchor probe can provide protection against bisulfite conversion of cytosines in the hybridized regions to uracils. Furthermore, since bisulfite treatment can convert non-methylated cytosine to uracil, the protection against conversion of cytosines to uracils at the TS area can allow for the use of amplification primers designed to anneal to the non-bisulfite converted DNA. For the pre-bisulfite conversion capture, the probe can also be designed against the unconverted sequence. Probes and primers that anneal to unconverted cytosines can be more straightforward to design and provide better hybridization.
In some cases, the enzymatic treatment can be performed for the methylation analysis. The enzyme can be methylation-sensitive or methylation dependent enzymes. The enzymes can be restriction enzymes. The enzymes can be methylation-sensitive restriction endonucleases. In other cases, the methylation analysis can be done by using specific antibodies or proteins that specifically bind to methylation sites to enrich methylated nucleic acids.

### a. Methylation treatment or enrichment after hybridization capture of a template nucleic acid

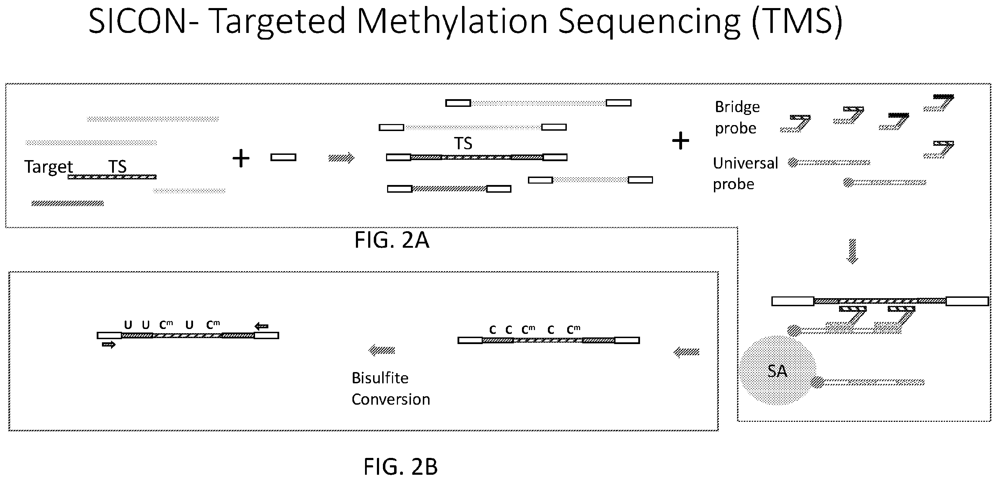

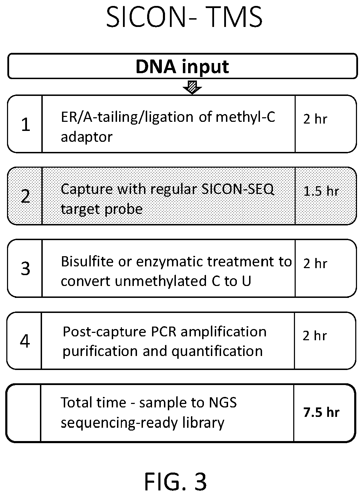

A template nucleic acid (e.g., DNA) can be used for synergistic, indirect hybridization and subsequent sequencing (SICON-SEQ) as described herein (see e.g., **FIG. 3****).** The template nucleic acid (e.g., DNA) can be, e.g., genomic DNA, or cfDNA. A template nucleic acid (e.g., DNA) can be directly hybridized to a capture probe or indirectly bound to adaptor anchor probe (or universal anchor probe) by bridge probe hybridization, e.g., as described herein, e.g., as illustrated in **FIGS. 1** **and** **2A****.** The hybridization captured template nucleic acid (e.g., DNA) can be treated with bisulfite, extended, and amplified subsequently **(****FIG. 2B****),** e.g., for targeted methylation sequencing (SICON-TMS). In some cases, the captured template nucleic acid can be treated with methylation-sensitive enzymes. In another case, the methylated nucleic acids of the captured template nucleic acid molecule can be enriched by specifically binding to antibodies or proteins that target methylated CpG sites in the template nucleic acid molecule. SICON-TMS can be compatible clinical samples with over a large range of nucleic material amount. In some cases, SICON-TMS can be used sequence samples with nucleic acid molecules of less than 5 ng, less than 4 ng, less than 3 ng, less than 2 ng, or less than 1 ng.

The target specific sequence or target specific region (TSR) of a capture probe or a bridge probe can be designed based on the target sequence of the template nucleic acid molecule, and the target sequence of the template nucleic acid molecule can retain non-methylated cytosine after the bisulfite treatment.

In some cases, the bisulfite treatment can occur before detachment of a target specific sequence of the bridge probe. The unmethylated cytosines in the TS and TSR sites can be protected from conversion to uracil during bisulfite treatment that occurs after hybridization of the TS and TSR of the capture probe or bridge probe to the template. Subsequently, the hybridized template can be treated with bisulfite during which the non-methylated cytosines in the hybridized TSR-TS region are not converted to uracil, whereas a non-methylated cytosine in the single stranded area is converted to uracil. The protection against conversion of cytosines to uracils at the TS area can allow for the use of probes designed to anneal to the non-bisulfite converted DNA.

In some cases, the bisulfite treatment can be performed after detachment of the capture probe or the bridge probe from the template nucleic acid sequence. The one or more cytosine residues in a primer binding site (e.g., an adaptor and/or in a template) may not protected from bisulfite conversion. Following bisulfite conversion, a primer binding site in an adaptor can comprise one or more uracils. A primer can be designed to be complementary to the adaptor sequence comprising one or more uracils. The primer can be 100% complementary to the adaptor sequence comprising one or more uracils, or less than 100% complementary to the adaptor sequence comprising one or more uracils.

A template can comprise one or more uracils after bisulfite treatment. A primer annealing to an adaptor can use the template comprising the one or more uracils for strand extension. The extended strand can comprise one or more adenines that are base-paired to the one or more uracils. The extension product can be denatured from the template. A primer can be annealed to the extension product in the region comprising the one or more adenines and extended. The primer can be used in amplification of the template with, e.g., an adaptor primer.

The methylation treatment or enrichment can be applied to the template nucleic acid molecules before the attachment of the adaptors. The methylation treatment or enrichment can be applied to the template nucleic acid molecules after the attachment of the adaptor. The methylation treatment or enrichment can be applied to the template nucleic acid molecules after the attachment of the first adaptor to the template. The methylation treatment or enrichment can be applied to the template nucleic acid molecules after the attachment of the second adaptor to the template.

### b. Methylation treatment or enrichment before hybridization capture of a template nucleic acid

Template nucleic acid molecules can be bisulfite treated prior to hybridization to capture probes or bridge probes. DNA can be treated with bisulfite to convert unmethylated cytosines to uracils. The bisulfite treated DNA can be used as an input for synergistic, indirect hybridization and subsequent sequencing (SICON-SEQ). The TSR of a probe can be designed to anneal to the template in which existing non-methylated cytosines have been converted to uracil. Following the hybridization capture, extension can be performed followed by target amplification. In some cases, the captured template nucleic acid can be treated with methylation-sensitive enzymes. In another case, the methylated nucleic acids of the captured template nucleic acid molecule can be enriched by specifically binding to antibodies or proteins that target methylated CpG sites in the template nucleic acid molecule.

The methylation treatment or enrichment can be performed to the template nucleic acid molecules before the attachment of the adaptors. The methylation treatment or enrichment can be applied to the template nucleic acid molecules after the attachment of the adaptor. The methylation treatment or enrichment can be applied to the template nucleic acid molecules after the attachment of the first adaptor to the template. The methylation treatment or enrichment can be applied to the template nucleic acid molecules after the attachment of the second adaptor to the template.

### III. Solid Phase Extraction

Methods are provided herein to select for templates that are hybridized to a bridge probe (or templates associated with an adaptor anchor probe via a bridge probe), e.g., before the adaptor anchor probe is ligated to the template. The methods can employ solid phase extraction. Methods are provided herein to bind a bridge probe, or adaptor anchor probe to a solid support. Suboptimal specificity can be introduced by the possibility that the adaptor anchor probe attaches (e.g., ligates) to the template independent of bridge probe. To reduce such non-specific ligation products as well as unbound probe, labels (e.g., biotin) and capture moieties (e.g., streptavidin beads) can be utilized.

The bridge probe, or adaptor anchor probe can comprise a label. The disclosed methods can further comprise capturing to the bridge probe, the adaptor anchor probe, or the hybridization complex comprising template nucleic acid molecule, bridge probe, and adaptor anchor probe by the label. The label can be biotin. The label can be a nucleic acid sequence, such as poly A or Poly T, or specific sequence. The nucleic acid sequence can be about 5 to 30 bases in length. The nucleic acid sequence can comprise DNA and/or RNA. The label can be at the 3' end of the bridge probe, or adaptor anchor probe. The label can be a peptide, or modified nucleic acid that can be recognized by antibody such as 5-Bromouridine, and biotin. The label can be conjugated to the bridge probe, or adaptor anchor probe by reactions such as "click" chemistry. "Click" chemistry can allow for the conjugation of a reporter molecule like fluorescent dye to a biomolecule like DNA. Click Chemistry can be a reaction between and azide and alkyne that can yield a covalent product (e.g., 1,5-disubstituted 1,2,3-triazole). Copper can serve as a catalyst.

The label can be captured on a solid support. The solid support can be magnetic. The solid support can comprise a bead, flow cell, glass, plate, device comprising one or more microfluidic channels, or a column. The solid support can be a magnetic bead.

The solid support (e.g., bead) can comprise (e.g., by coated with) one or more capture moieties that can bind the label. The capture moiety can be streptavidin, and the streptavidin can bind biotin. The capture moiety can be an antibody. The antibody can bind the label. The capture moiety can be a nucleic acid, e.g., a nucleic acid comprising DNA and/or RNA. The nucleic acid capture moiety can bind a sequence on, e.g., an adaptor anchor probe or bridge probe. In some cases, an anti-RNA/DNA hybrid antibody bound to a solid surface can be used as a capture moiety.

The label and the capture moiety can bind through one or more covalent or non-covalent bonds. Following capture of the bridge probe, adaptor anchor probe, or the hybridization complex on the solid support, the solid support can be washed to remove, e.g., unbound template from the sample. In some cases, no wash step is performed. The wash can be stringent or gentle. The captured bridge probe or adaptor anchor probe that are hybridized to template nucleic acid molecule can be eluted, e.g., by adding free biotin to the sample when the label is biotin and the capture moiety is streptavidin.

Extension steps (e.g., extension of an adaptor primer that anneals to an adaptor) can be performed while the bridge probe or adaptor anchor probe are captured on a solid support or after elution of the bridge probe (and hybridized template) or adaptor anchor probe (and indirectly hybridized template) are eluted from the solid support.

Cleanups can be performed using streptavidin beads after template, bridge probe, and adaptor anchor probe hybridization, wherein the 3' end of the adaptor anchor probe is biotinylated. Both the hybridization complex and the free adaptor anchor adaptor can bind to the bead. The unbound template and bridge probe can be washed away. The 5' end or the 3' end of a first and or second bridge probe can be biotinylated. Streptavidin beads can be used to remove the unhybridized adaptor anchor adaptor and template, which can prevent random ligation of an adaptor anchor probe and a template.

### IV. Template nucleic acid molecules

The template nucleic acid can be DNA or RNA. The DNA can be genomic DNA (gDNA), mitochondrial DNA, viral DNA, cDNA, cfDNA, or synthetic DNA. The DNA can be double-stranded DNA, single-stranded DNA, fragmented DNA, or damaged DNA. RNA can be mRNA, tRNA, rRNA, microRNA, snRNA, piRNA, small non-coding RNA, polysomal RNA, intron RNA, pre-mRNA, viral RNA, or cell-free RNA.

The template nucleic acid can be naturally occurring or synthetic. The template nucleic acid can have modified heterocyclic bases. The modification can be methylated purines or pyrimidines, acylated purines or pyrimidines, alkylated riboses, or other heterocycles. The template nucleic acid can have modified sugar moieties. The modified sugar moieties can include peptide nucleic acid. The template nucleic acid can comprise peptide nucleic acid. The template nucleic acid can comprise threose nucleic acid. The template nucleic acid can comprise locked nucleic acid. The template nucleic acid can comprise hexitol nucleic acid. The template nucleic acid can be flexible nucleic acid. The template nucleic acid can comprise glycerol nucleic acid.

The template nucleic acid molecule can be captured and enriched from low-input (e.g. 1 ng of nucleic acid materials) samples such as cell-free DNA (cfDNA) and circulating tumor DNA (ctDNA). The low-input samples can have 1 ng, 2 ng, 3 ng, 4 ng, 5 ng, 6 ng, 7 ng, 8 ng, 9 ng, 10 ng, or more of nucleic acid materials. The low-input samples can have less than 10 ng, 9 ng, 8 ng, 7 ng, 6 ng, 5 ng, 4 ng, 3 ng, 2 ng, 1 ng, or less of nucleic acid materials. The low-input samples can have from 200 pg to 10 ng of nucleic acid materials. The low-input samples can have less than 10 ng of nucleic acid materials. The low-input sample can less than 10 ng, 5 ng, 1 ng, 100 pg, 50 pg, 25 pg, or less of the nucleic acid materials. In some cases, the input samples can have 1 ng, 10 ng, 20 ng, 30 ng, 40 ng, 50 ng, or more of nucleic acid molecule. The input samples can have less than 50 ng, 40 ng, 30 ng, 20 ng, 10 ng, 1 ng, or less of nucleic acid materials. The capture and enrichment can be done by target probe hybridization. The target probe can be capture probe, bridge probe, and/or adaptor anchor probe. The target probe can comprise one or more binding moieties. The binding moiety can be a biotin. The binding moieties can be attached to a support. The support can be a bead. The bead can be a streptavidin bead.

The template nucleic acid can be damaged. The damaged nucleic acid can comprise altered or missing bases, and/or modified backbone. The template nucleic acid can be damaged by oxidation, radiation, or random mutation. The template nucleic acid can be damaged by bisulfite treatment.

For damaged DNA, the present disclosure can eliminate double-strand DNA repair steps, providing higher conversion rate and improved sensitivity due to less DNA loss from fewer steps in the process.

Damaged dsDNA (with a nick) or ssDNA can be used as template for a library construction. For the damaged dsDNA, the dsDNA can be denatured so at least one undamaged strand can be used as a template. The template can then be hybridized and attached to a capture probe and amplified using various primers.

The template can be derived from cell-free DNA (cfDNA) or circulating tumor DNA (ctDNA). The cfDNA can be fetal or tumor in source. The template can be derived from liquid biopsy, solid biopsy, or fixed tissue of a subject. The template can be cDNA and can be generated by reverse transcription. The template nucleic acid can be derived from fluid samples, including not limited to plasma, serum, sputum, saliva, urine, or sweat. The fluid samples can be bisulfite treated to study the methylation pattern of the template nucleic acid and/or to determine the tissue origin of the template nucleic acid. The template nucleic acid can be derived from liver, esophagus, kidney, heart, lung, spleen, bladder, colon, or brain. The template nucleic acid can be treated with bisulfite to analyze methylation pattern of organ the template nucleic acid is derived from. The subject can suffer from methylation related diseases such as autoimmune disease, cardiovascular diseases, atherosclerosis, nervous disorders, and cancer.

The template nucleic acid can be derived from male or female subject. The subject can be an infant. The subject can be a teenager. The subject can be a young adult. The subject can be an elderly person.

The template nucleic acid can originate from human, rat, mouse, other animal, or specific plants, bacteria, algae, viruses, and the like. The template nucleic acid can originate from primates. The primates can be chimpanzees or gorillas. The other animal can be a rhesus macaque. The template also can be from a mixture of genomes of different species including host-pathogen, bacterial populations, etc. The template can be cDNA made from RNA expressed from genomes of two or more species.

The template nucleic acid can comprise a target sequence. The target sequence is an exon. The target sequence is can be an intron. The target sequence can comprise a promoter. The target sequence can be previously known. The target sequence can be partially known previously. The target sequence can be previously unknown. The target sequence can comprise a chromosome, chromosome arm, or a gene. The gene can be gene associated with a condition, e.g., cancer. The template nucleic acid molecule can be dephosphorylated before hybridization to, e.g, reduce the rate of self-ligation.

### V. Bridge Probes

Bridge probe are used to hybridize a template nucleic acid molecule with target sequence and an adaptor anchor probe. The bridge probe can further allow indirect association an adaptor anchor probe and template and thereby facilitating their attachment. The ligation rate of a free adaptor anchor probe and template can be very low because of the randomness of the interaction. But a hybridized bridge probe can increase the probability of ligation between adaptor anchor probe and a template compared to that with a free adaptor anchor probe. The bridge probes comprise DNA or RNA. The bridge probe can comprise of uracil and methylated cytosine. The bridge probe might not comprise of uracil.

The bridge probe comprises target specific region (TSR) that hybridizes to target sequence. The bridge probe comprise adaptor landing sequence (ALS) that hybridizes to bridge binding sequence of adaptor anchor probe. The bridge probe can comprise a linker connecting TSR and ALS. The TSR can be located in the 3'-portion of the bridge probe. The TSR can be located in the 5'-portion of the bridge probe.

The bridge probe can comprise one or more molecular barcodes. The bridge probe can comprise one or more binding moieties. The binding moiety can be a biotin. The binding moieties can be attached to a support. The support can be a bead. The bead can be a streptavidin bead.

The bridge probe can comprise about 400 nucleotides, about 300 nucleotides, about 200 nucleotides, about 120 nucleotides, about 100 nucleotides, about 90 nucleotides, about 80, about 70 nucleotides, about 50 nucleotides, about 40 nucleotides, about 30 nucleotides, about 20 nucleotides, or about 10 nucleotides.

Multiple bridge probes can be used to anneal to multiple target sequences in a sample. The bridge probes can be designed to have similar melting temperatures. The melting temperatures for a set of bridge probes can be within about 15°C, within about 10oC, within about 5°C, or within about 2°C. The melting temperature for one or more bridge probes can be about 75°C, about 70°C, about 65°C, about 60°C, about 55°C, about 50°C, about 45°C, or about 40°C. The melting temperature for the bridge probe can be about 40°C to about 75°C, about 45°C to about 70°C, 45°C to about 60°C, or about 52°C to about 58°C.

Use of an adaptor anchor probe along with one or more bridge probe around a particular bridge probe can help to stabilize the hybridization of the particular bridge probe to the its target sequence through synergistic effect. A hybridization temperature to form the multiple bridge probe assembly can be higher than the melting temperature of a single bridge probe. The higher temperature can result in a better capture specificity by reducing nonspecific hybridization that can occur at lower temperature. The hybridization temperature can be about 5°C, about 10°C, about 15°C, or about 20°C higher than the melting temperature of individual bridge probe. The hybridization temperature can be about 5°C to about 20°C higher than the melting temperature of a bridge probe, or about 5°C to about 20°C higher than an average melting temperature of a plurality of bridge probes.

The hybridization temperature for multiple bridge probes can be about 75°C, about 70°C, about 65°C, about 60°C, about 55°C, or about 50°C. The hybridization temperature for multiple bridge probes can be about 50°C to about 75°C, 55°C to about 75°C, 60°C to about 75°C, or 65°C to about 75°C.

The bridge probe can further comprise a label. The label can be fluorescent. The fluorescent label can be organic fluorescent dye, metal chelate, carbon nanotube, quantum dot, gold particle, or fluorescent mineral. The label can be radioactive. The label can be biotin. The bridge probe can bind to labeled nucleic acid binder molecule. The nucleic acid binder molecule can be antibody, antibiotic, histone, antibody, or nuclease.

The bridge probe can comprise a linker. The linker can comprise about 30 nucleotides, about 25 nucleotides, about 20 nucleotides, about 15 nucleotides, about 10 nucleotides, or about 5 nucleotides. The linker can comprise about 5 to about 20 nucleotides.

The linker can comprise non-nucleic acid polymers (e.g., string of carbons). The linker non-nucleotide polymer can comprise about 30 units, about 25 units, about 20 units, about 15 units, about 10 units, or about 5 units.

The bridge probe can be blocked at the 3' and/or 5' end. The bridge probe can lack a 5' phosphate. The bridge probe can lack a 3' OH. The bridge probe can comprise a 3'ddC, 3'inverted dT, 3'C3 spacer, 3' amino, or 3' phosphorylation.

### VI. Adaptor Anchor Probe

The adaptor anchor probe or universal anchor probe comprises one or more bridge binding sequences that hybridize to adaptor landing sequence of the two or more bridge probes.

The adaptor anchor probe can comprise spacers in between the BBSs. The presence of the one or more spacers can improve the efficiency of the hybridization capture and increase the specificity of the capture.

The adaptor anchor probe can comprise a molecular barcode (MB). The adaptor anchor probe can comprise a bridge binding sequence (BBS) to which the one or more bridge probes can hybridize to. The adaptor anchor probe can comprise from 1to100 BBSs. The adaptor anchor probe can comprise an index for distinguishing samples. The molecular barcode or index can be 5' of the adaptor sequence and 5' of the BBS.

The adaptor anchor probe can comprise about 400 nucleotides, about 200 nucleotides, about 120 nucleotides, about 100 nucleotides, about 90 nucleotides, about 80 nucleotides, about 70 nucleotides, about 50 nucleotides, about 40 nucleotides, about 30 nucleotides, about 20 nucleotides, or about 10 nucleotides. The adaptor anchor probe can be about 20 to about 70 nucleotides.

The melting temperature of adaptor anchor probe to the bridge probe can be about 65°C, about 60°C, about 55°C, about 50°C, about 45°C. or about 45°C to about 70°C.

The adaptor anchor probe can comprise a label. The label can be fluorescent. The fluorescent label can be an organic fluorescent dye, metal chelate, carbon nanotube, quantum dot, gold particle, or fluorescent mineral. The label can be radioactive. The label can be biotin. The adaptor anchor probe can bind to labeled nucleic acid binder molecule. The nucleic acid binder molecule can be antibody, antibiotic, histone, antibody, or nuclease.

### VII. Adaptors/Adaptor primers

One or more adaptors can be attached to a plurality of template nucleic acids for construction of a library. The library can be new-generation sequencing (NGS) library. One adaptor can be attached to a 5' end or 3' end of a template nucleic acid molecule. Two adaptors can be attached to a 5' end and a 3' end of a template nucleic acid molecule. The one or more adaptors can be attached to the template nucleic acids by ligation. The attachment of the one or more adaptors can be performed prior to hybridization of the template nucleic acid and target probes. In some cases, adaptors can be added the captured template nucleic acid post-hybridization. The one or more adaptors can comprise a molecular barcode (MB).

One or more adaptor primers can be hybridized to the one or more adaptors attached to the template nucleic acid molecules. In some cases, adaptors are incorporated in adaptor anchor probes or capture probes. In certain cases, Attached, added, or incorporated adaptors can provide sites for primer hybridization for amplification. A first adaptor (AD1) can be attached to the template via a capture probe or an adaptor anchor probe. A primer against AD1 can be utilized to synthesize a strand complementary to the template. A second adaptor (AD2) can be attached to 5' end of template and/or 3' end of the complementary strand to further amplify the template. A library can be constructed using AD1 primer and AD2 primer. Selective amplification can be performed using AD1 primer and primer against TSR or its flanking regions.

The adaptor can be a single-stranded nucleic acid. The adaptor can be double-stranded nucleic acid. The adaptor can be partial duplex, with a long strand longer than a short strand, or with two strands of equal length.

### VIII. Enzymes

Examples of DNA polymerases that can be used in the methods described herein include Klenow polymerase, Bst DNA polymerase, Bca polymerase, phi 29 DNA polymerase, Vent polymerase, Deep Vent polymerase, Taq polymerase, T4 polymerase, T7 polymerase, or E. coli DNA polymerase 1.

Examples of ligases that can be used in the methods described herein include CircLigase, CircLigase II, E. coli DNA ligase, T3 DNA ligase, T4 DNA ligase, T7 DNA ligase, DNA ligase I, DNA ligase II, DNA ligase III, DNA ligase IV, Taq DNA ligase, or Tth DNA ligase.

Examples of methylation-sensitive or methylation-dependent restriction enzyme that can be used in the methods described herein include Aat II, Acc II, Aor13H I, Aor51H I, BspT104 I, BssH II, Cfr10 I, Cla I, Cpo I, Eco52 I, Hae II, Hap II, Hha I, Mlu I, Nae I, Not I, Nru I, Nsb I, PmaC I, Psp1406 I, Pvu I, Sac II, Sal I, Sma I, and SnaB I.

### IX. Downstream Analysis of Amplification Products

The amplified products generated using methods described herein can be further analyzed using various methods including southern blotting, polymerase chain reaction (PCR) (e.g., real-time PCR (RT-PCR), digital PCR (dPCR), droplet digital PCR (ddPCR), quantitative PCR (Q-PCR), nCounter analysis (Nanostring technology), gel electrophoresis, DNA microarray, mass spectrometry (e.g., tandem mass spectrometry, matrix-assisted laser desorption ionization time of flight mass spectrometry (MALDI-TOF MS), chain termination sequencing (Sanger sequencing), or next generation sequencing.

The next generation sequencing can comprise 454 sequencing (ROCHE) (using pyrosequencing), sequencing using reversible terminator dyes (ILLUMINA sequencing), semiconductor sequencing (THERMOFISHER ION TORRENT), single molecule real time (SMRT) sequencing (PACIFIC BIOSCIENCES), nanopore sequencing (e.g., using technology from OXFORD NANOPORE or GENIA), microdroplet single molecule sequencing using pyrophosphorolyis (BASE4), single molecule electronic detection sequencing, e.g., measuring tunnel current through nanoelectrodes as nucleic acid (DNA/RNA) passes through nanogaps and calculating the current difference (QUANTUM SEQUENCING from QUANTUM BIOSYSTEMS), GenapSys Gene Electomic Nano-Integrated Ultra-Sensitive (GENIUS) technology (GENAPYS), GENEREADER from QIAGEN, sequencing using sequential hybridization and ligation of partially random oligonucleotides with a central determined base (or pair of bases) identified by a specific fluorophore (SOLiD sequencing). The sequencing can be paired-end sequencing.

The number of target sequences from a sample that can be sequenced using methods described herein can be about 5, 10, 15, 25, 50, 100, 1000, 10,000, 100,000, or 1,000,000, or about 5 to about 100, about 100 to about 1000, about 1000 to about 10,000, about 10,000 to about 100,000, or about 100,000 to about 1,000,000.

Nucleic acid libraries generated using methods described herein can be generated from more than one sample. Each library can have a different index associated with the sample. For example, a capture probe or an adaptor anchor probe can comprise an index that can be used to identify nucleic acids as coming from the same sample (e.g., a first set of capture probes or adaptor anchor probes comprising the same first index can be used to generate a first library from a first sample from a first subject, and a second set of capture probes or adaptor anchor probes comprising the same second index can be used to generate a second library from a second sample from a second subject, the first and second library can be pooled, sequenced, and an index can be used to discern from which sample a sequenced nucleic acid was derived). Amplified products generated using the methods described herein can be used to generate libraries from at least 2, 5, 10, 25, 50, 100, 1000, or 10,000 samples, each library with a different index, and the libraries can be pooled and sequenced, e.g., using a next generation sequencing technology.

The sequencing can generate at least 100, 1000, 5000, 10,000, 100,000, 1,000,000, or 10,000,000 sequence reads. The sequencing can generate between about 100 sequence reads to about 1000 sequence reads, between about 1000 sequence reads to about 10,000 sequence reads, between about 10,000 sequence reads to about 100,000 sequence reads, between about 100,000 sequence reads and about 1,000,000 sequence reads, or between about 1,000,000 sequence reads and about 10,000,000 sequence reads.

The depth of sequencing can be about 1x, 5x, 10x, 50x, 100x, 1000x, or 10,000x. The depth of sequencing can be between about 1x and about 10x, between about 10x and about 100x, between about 100x and about 1000x, or between about 1000x and about 10000x.

### X. Bioinformatics Analysis

Provided herein are methods for the bioinformatic analysis of sequencing data. For example, methods of excluding molecules with incomplete bisulfite conversion, and methods of analyzing methylation patterns in samples with very low disease molecule content.

### a. Exclusion of molecules with incomplete bisulfite conversion

A filtering technique to exclude molecules with incomplete C>T conversions is used to enhance the robustness of the molecule count and methylation fraction data.

Sequencing reads mapped to each differentially methylated region (DMR) can be de-duplicated using read start and end nucleotide location in the genome and unique molecular identifier information. De-duplication can also be done with start and end location information alone at a lower accuracy.

The de-duplicated reads are filtered according to the number of unconverted C's in the CH context, where C represents a cytosine, and H represents any of the three nucleotides: C (cytosine), A (Adenine) or T (thymine). The existence of C's in CH context that are not converted to T indicates a high likelihood of incomplete bisulfite or enzymatic treatment of the molecule. When the number of unconverted C's in the CH context is greater than a preset threshold, the read is discarded. In some cases, the threshold number of unconverted C's in the CH context is 1, 2, 3, 4, 5, 6, 7, 8, 9, or 10. In some cases, a read may be discarded if the percentage of unconverted C's in the CH context (as a percent of the total number of C's in the CH context) is greater than 1%, 2%, 3%, 4%, 5%
, 6%, 7%, 8%, 9%, 10%, 12%, 14%, 16%, 18%, 20%, 25%, 30%, 35%, 40%, or 50%.

### b. SICON TMS analysis

Current methods for the analysis of methylation sequencing data may involve calculating either or both of two metrics for down-stream analysis: (1) the methylation fractions at individual CpG sites; (2) the methylation density of genomic regions of interest. For (1), the number of methylated C's at a CpG site may be divided by the total number of molecules covering the CpG site. For (2), an average of all methylation fractions of CpG sites in the defined genomic region may be calculated. As a slight modification to the concepts above, methylation haplotype load (MHL) may be introduced in an effort to take into account the differences in methylation patterns in molecules of a region. In essence, MHL represents an average measure across an admixture of molecules, with weights added to account for block lengths. These methods take an average measure across DNA molecules in all of the molecules sequenced, including both disease-derived and healthy normal-derived materials.

In tissue sequencing data, taking an average across all molecules is usually an adequate and necessary approach. For example, in the case of tumor biopsy tissues, the tumor content may be moderately high (e.g. 20% or more). A significant difference in methylation level between tumor and normal tissues could be reflected in the averages of tumor-normal mixed tissue and the averages of pure normal tissue. The average is often performed out of necessity because most bisulfite sequencing data have a low complexity at each genomic region. For example, 30x may be considered deep coverage in whole genome bisulfite sequencing and many studies have much lower coverage. An average across many CpG sites in the region smooths out variability due to low coverage and may enhance the robustness of the measurements. In the context of samples with very low disease molecule content such as liquid biopsy using plasma cfDNA from a tumor patient, where the tumor content is often below 0.1%, an average across an admixture of healthy normal and disease-derived molecules may be dominated by normal molecules. In other words, the tumor-derived methylation information is overwhelmed by the normal-derived molecules in the action of taking an average.

A method to analyze methylation sequencing data is described here as "SICON TMS analysis". Briefly, the number of CpG sites on each sequenced molecule is counted, and the methylation fraction of these sites is calculated. The data pair, consisted of a CpG count and a methylation fraction, represents one data point in the downstream classification model. Compared to the average-based methods, no average of methylation information from disease-derived and normal-derived molecules is performed. The methylation profile of disease-derived and normal-cell-derived molecules may thus be kept separate. Each of the resulting reads may contain the CpG methylation information from a unique DNA molecule captured by the assay. Two metrics are collected from each read:
1) N: the total number of CpGs in the read;
2) M: the number of methylated CpGs in the read.
From 1) and 2), a third metric is calculated as:
3) f = M/N, the fraction of CpGs that are methylated in the current read.

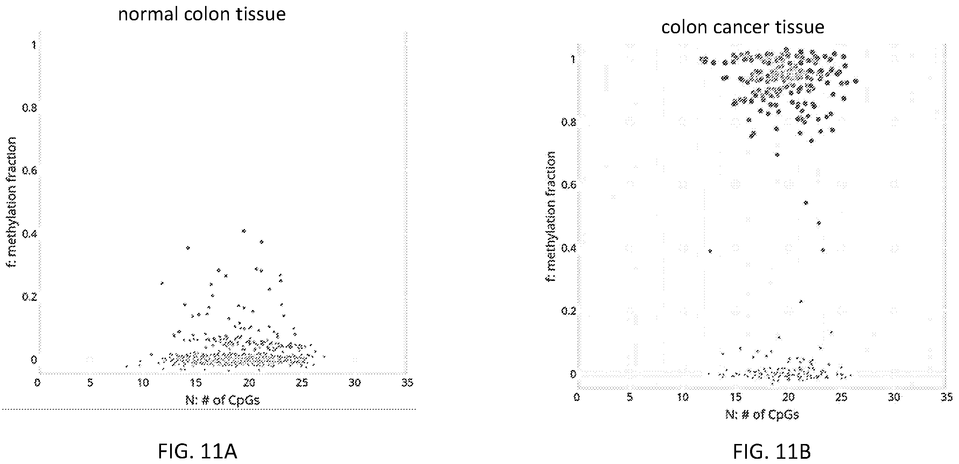

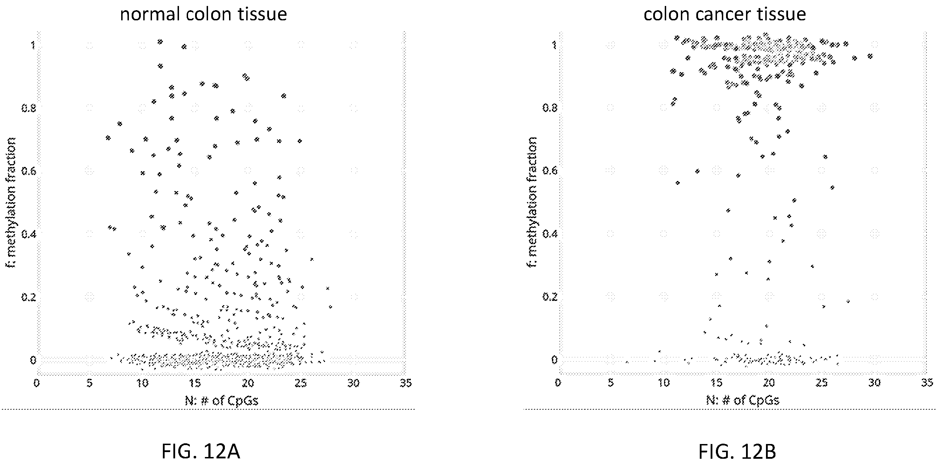

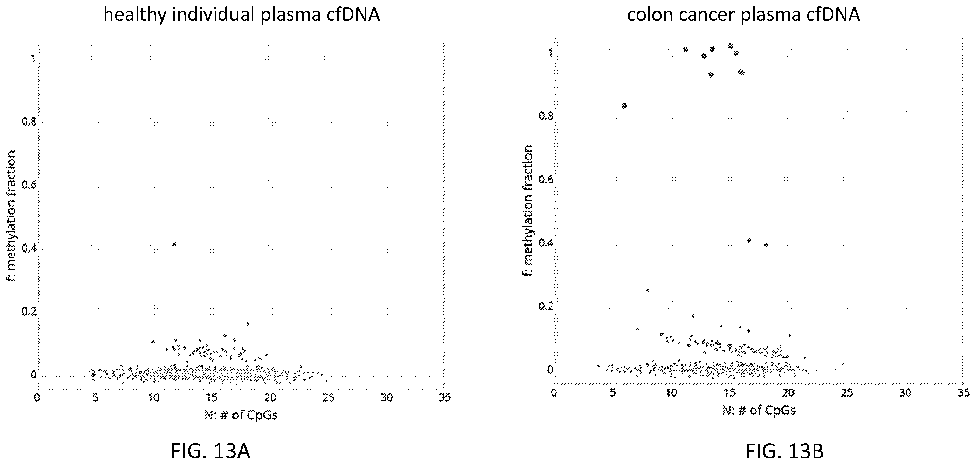

The data pairs (N, f) are collected for each of the molecules on all DMRs in the assay. A scatter plot showing f (y axis) vs N (x axis) can be generated for a DMR, with every read in the DMR shown as a dot in the plot. For example, FIG. 11 shows the molecule methylation scatter pattern of DMR1 in a normal colon tissue (FIG. 11A) and a colon cancer tissue genomic DNA (FIG. 11B). It demonstrates a DMR where there is no hyper-methylated DNA molecule in normal colon tissue and a large amount of hyper-methylated molecules in colon cancer tissue. FIG.12A and 12B show the molecule methylation scatter pattern of DMR2 in a normal colon tissue and a colon cancer tissue genomic DNA respectively. It demonstrates a DMR where there are some hyper-methylated DNA molecules in normal colon tissue (FIG. 12A) and a larger amount of hyper-methylated molecules in colon cancer tissue (FIG. 12B). FIG. 13 shows the molecule methylation scatter pattern of DMR1 and DMR2 in plasma cfDNA from a healthy individual (FIG. 13A) and a colon cancer patient (FIG. 13B). The counts of hyper-methylated molecules illustrated in the upper part of FIG. 13B from each DMR are the basis for disease detection from liquid biopsy.

Several further analyses can be conducted. For example a filter can be applied to count hyper-methylated molecules. Filter for hyper-methylated molecules: a threshold f0 may be selected to count all molecules with f>f0 (i.e. in the upper part of the scatter plot). These reads are hyper-methylated reads that are a signature of the disease tissue (such as colon cancer). The hyper-methylation filter threshold (f0) may be set at 0.2, 0.3, 0.4, 0.5, 0.6, 0.7, 0.8, or 0.9. In some cases, the hyper-methylation filter threshold (f0) may be set based on the analysis of methylation in normal tissue, or a sample from a healthy subject. For example, the hyper-methylation filter threshold (f0) may be set as 0.5, 1, 1.5, 2, 2.5, or 3 standard deviations from the mean methylation fraction in a normal tissue sample, or a sample from a healthy subject.

Molecules may also be filtered for robust signal. Filter for molecules with a robust signal: an additional threshold N0 may be selected to keep only reads with N>N0 to enhance the robustness of the molecule count. The threshold N0 may be set at 1, 2, 3, 4, 5, 6, 7, 8, 9, 10, 11, 12, 13, 14, 15, 16, 17, 18, 19, 20, 21, 22, 23, 24, 25, 26, 27, 28, or 30.

Filtering for hypermethylated molecules and robust signal may ensure that only the robust hyper-methylated molecules are counted for each DMR. This may improve the quality of analysis, and/or the sensitivity.

In some cases, the threshold values f0 and N0 are the same through all DMRs. In some cases, the thresholds values f0 and N0 may be customized for each individual DMR. In some cases, the threshold value f0 may be the same through all DMRs and the thresholds N0 may be customized for each individual DMR. In some cases, the threshold value N0 may be the same through all DMRs and the threshold f0 may be customized for each individual DMR. In some cases, both thresholds f0 and N0 may be customized for each individual DMR
The robust hyper-methylated molecule counts across all DMRs in the assay may be fed into a model to determine disease status of the sample using machine learning classifier methods.

### XI. Sequential target enrichment

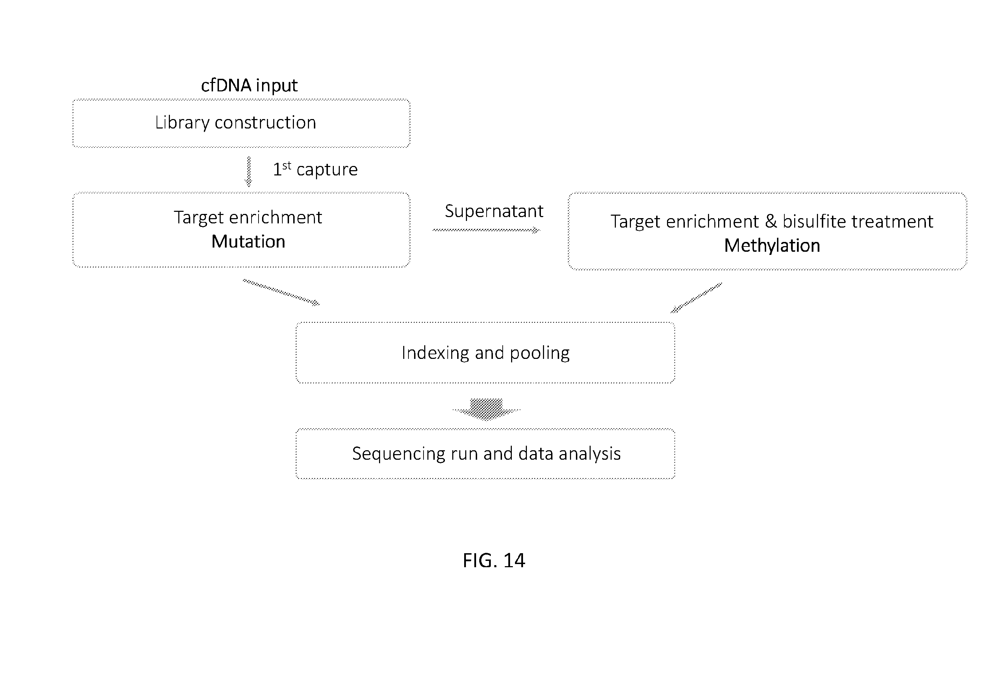

The present disclosure provides a method of sequential hybridization-based enrichment which may be used to enrich for two or more panels of sequences from the same DNA input without splitting. FIG. 14 illustrates a method of performing sequential enrichment. In some cases, a method of sequential enrichment may comprise obtaining a sample comprising a plurality of nucleic acid molecules and performing a first target enrichment to enrich for nucleic acid molecules comprising sequences corresponding to a first panel of one or more genome regions, thereby generating a first enriched sample comprising nucleic acids enriched for sequences corresponding to the first panel of one or more genome regions. The first target enrichment may also generate a remaining sample (or a first remaining sample) comprising nucleic acids depleted for sequences corresponding to the first panel of one or more genome regions. This remaining sample may be used for performing a second target enrichment upon the remaining sample to enrich for nucleic acid molecules comprising sequences corresponding to a second panel of one or more genome regions, thereby generating a second enriched sample comprising nucleic acids enriched for sequences corresponding to the second panel of one or more genome regions. The first panel of one or more genome regions and the second panel of one or more genome regions are generally different. In some cases, third, fourth, or further rounds of target enrichment may be performed with third, fourth or further panels of genome regions.

For example, a panel of one or more genome regions may comprise a panel of 1-50,000, 5-10000, or 5-5000 genome regions associated with mutation hotspots, oncogenes, tumor suppressor genes, oncogene exons, tumor suppressor exons, or regulatory regions. In another example, a panel of one or more genome regions may comprise a panel of 5-5000 genome regions associated with differentially methylated regions, with epigenetic modifications, with introns, with promoters, or with other regulatory sequences. In some cases, a panel comprises 50-500 genome regions associated with hypermethylation in cancer.

Because Point-n-Seq is a pre amplification and pre conversion enrichment technology The enriched samples may be analyzed by sequencing, or may be bisulfide treated (or enzymatically treated) prior to sequencing to assess methylation. In some cases, a first enriched sample may be analyzed by sequencing to assess mutations while a second enriched sample is bisulfide ( or enzymatical) treated prior to sequencing to assess methylation. In some cases, a first enriched sample and a second enriched sample are both assessed by straightforward sequencing to access genomic alteration, however the samples may be sequenced at different depths. In some cases, an analysis of a first enriched sample may be performed prior to performing a second target enrichment step. The results of the analysis of the first enriched sample may be used to select a second panel for the second enrichment step.

The target enrichment comprises hybridizing a first target specific region of a first bridge probe to a first target sequence of a molecule with a sequence corresponding to the genome region, wherein a first adaptor landing sequence of the first bridge probe is bound to a first bridge binding sequence of an adaptor anchor probe; and hybridizing a second target specific region of a second bridge probe to a second target sequence of the molecule with a sequence corresponding to the genome region, wherein a second adaptor landing sequence of the second bridge probe is bound to a second bridge binding sequence of the adaptor anchor probe. As described herein the anchor probe may comprise a binding moiety. The method generally comprises attaching adaptors to the 5' end or the 3' ends of nucleic acid molecules of the plurality of nucleic acid molecules, thereby generating a library of nucleic acid molecules comprising adaptors.

The sequential target enrichment described herein may be highly efficient. For example, when a second enriched sample is bisulfite treated and subjected to a sequencing reaction the number of informative reads of the sequencing reaction may be at least 60%, 65%, 70%, 75%, 80%, or 85% of the number of informative reads that could be obtained from the sample if it was subjected to a single target enrichment to enrich for nucleic acid molecules comprising sequences corresponding to a second panel of one or more genome regions.

The sequential target enrichment methods described herein may be generalized to any nucleic sample. The methods may be particularly useful for analysis of limited nucleic acid samples.

### XII. Applications

### a. Detection of nucleic acid features

The amplified nucleic acid products generated using the methods described herein can be analyzed for one or more nucleic acid features. The one or more nucleic acid features can be one or more methylation events. The methylation can be methylation of a cytosine in a CpG dinucleotide. The methylated base can be a 5-methylcytosine. A cytosine in a non-CpG context can be methylated. The methylated or unmethylated cytosines can be in a CpG island. A CpG island can be a region of a genome with a high frequency of CpG sites. The CpG island can be at least 200 bp, or about 300 to about 3000 bp. The CpG island can be a CpG dinucleotide content of at least 60%. The CpG island can be in a promoter region of a gene. The methylation can be 5-hmC (5-hydroxymethylcytosine), 5-fC (5-formylcytosine), or 5-caC (5-carboxylcytosine). The methods described herein can be used to detect methylation patterns, e.g., of DNA from a solid tissue or from a biological fluid, e.g., plasma, serum, urine, or saliva comprising, e.g., cell-free DNA.

The one or more nucleic acid features can be a de novo mutation, nonsense mutation, missense mutation, silent mutation, frameshift mutation, insertion, substitution, point mutation, single nucleotide polymorphism (SNP), single nucleotide variant (SNV), de novo single nucleotide variant, deletion, rearrangement, amplification, chromosomal translocation, interstitial deletion, chromosomal inversion, loss of heterozygosity, loss of function, gain of function, dominant negative, or lethal mutation. The amplified nucleic acid products can be analyzed to detect a germline mutation or a somatic mutation. The one or more nucleic acid features can be associated with a condition, e.g., cancer, autoimmune disease, neurological disease, infection (e.g., viral infection), or metabolic disease.

### b. Diagnosis/detections/monitoring

The disclosed methods can also be used to diagnosis or detect a disease or condition. The disease or condition can be connected to methylation abnormalities. The condition can be a psychological disorder. The condition can be aging. The condition can be a disease. The condition (e.g., disease) can be a cancer, a neurological disease (e.g., Alzheimer's disease, autism spectrum disorder, Rett Syndrome, schizophrenia), immunodeficiency, skin disease, autoimmune disease (e.g., Ocular Behcet's disease, systemic lupus erythematosus (SLE), rheumatoid arthritis (RA), multiple sclerosis, infection (e.g., viral infection), or metabolic disease (e.g., hyperglycemia, hyperlipidemia, type 2 diabetes mellitus). The cancer can be, e.g., colon cancer, breast cancer, liver cancer, bladder cancer, Wilms cancer, ovarian cancer, esophageal cancer, prostate cancer, bone cancer, or hepatocellular carcinoma, glioblastoma, breast cancer, squamous cell lung cancer, thyroid carcinoma, or leukemia (see e.g., Jin and Liu (2018) DNA methylation in human disease. Genes & Diseases, 5:1-8). The condition can be Beckwith-Wiedemann Syndrome, Prader-Willi syndrome, or Angelman syndrome.

The methylation patterns of cell-free DNA generated using methods provided herein can be used as markers of cancer (see e.g., Hao et al., DNA methylation markers for diagnosis and prognosis of common cancers. Proc. Natl. Acad. Sci. 2017; international PCT application publication no. WO2015116837). The methylation patterns of cell-free DNA can be used to determine tissues of origin of DNA (see e.g., international PCT application publication no. WO2005019477). The methods described herein can be used to determine methylation haplotype information and can be used to determine tissue or cell origin of cell-free DNA (see e.g., Seioighe et al, (2018) DNA methylation haplotypes as cancer markers. Nature Genetics 50, 1062-1063; international PCT application publication no. WO2015116837; U.S. patent application publication no. 20170121767). The methods described herein can be used to detect methylation levels, e.g., of cell-free DNA, in subjects with cancer and subjects without cancer (see e.g., Vidal et al. A DNA methylation map of human cancer at single base-pair resolution. Oncogenomics 36, 5648-5657; international PCT application publication no. WO2014043763). The methods described herein can be used to determine methylation levels or to determine fractional contributions of different tissues to a cell-free DNA mixture (see e.g., international PCT application publication no. WO2016008451). The methods described herein can be used for tissue of origin of cell-free DNA, e.g., in plasma, e.g., based on comparing patterns and abundance of methylation haplotypes (see e.g., Tang et al., (2018) Tumor origin detection with tissue-specific miRNA and DNA methylation markers. Bioinformatics 34, 398-406; international PCT application publication no. WO2018119216). The methods described herein can be used to distinguish cancer cells from normal cells and to classify different cancer types according to their tissues of origin (see e.g., U.S. Patent Application Publication No. 20170175205A1). The methods provided herein can be used to detect fetal DNA or fetal abnormalities using a maternal sample (see e.g., Poon et al. (2002) Differential DNA Methylation between Fetus and Mother as a Strategy for Detecting Fetal DNA in Maternal Plasma. Clinical Chemistry, 48: 35-41).

The disclosed methods can be used for monitoring of a condition. The condition can be disease. The disease can be a cancer, a neurological disease (e.g., Alzheimer's disease), immunodeficiency, skin disease, autoimmune disease (e.g., Ocular Behcet's disease), infection (e.g., viral infection), or metabolic disease. The cancer can be in remission. Since the disclosed methods can use cfDNA and ctDNA to detect low level of abnormalities, the present disclosure can provide relatively noninvasive method of monitoring diseases. The disclosed methods can be used for monitoring a treatment or therapy. The treatment or therapy can be used for a condition, e.g., a disease, e.g., cancer, or for any condition disclosed herein.
The methods described herein may allow for enrichment of target molecules directly from cfDNA before bisulfite conversion and amplification. The methods may also enable development of small, focused, panels that interrogate the methylation status of 1 to ~1000 markers for a given disease. In some cases, a kit may be produced for a panel that interrogates the methylation status of 1 to about 10000 differentially methylated regions for a given disease.

### EXAMPLES

### Example 1

### Capture by synergistic indirect hybridization

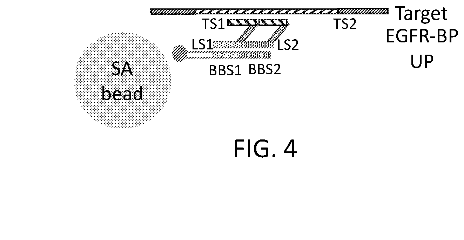

A synergistic indirect capture of nucleic acid for sequencing (SICON-SEQ) experiment was carried out with two bridge probes with different sequences and an adaptor anchor probe/universal anchor probe (UP, SEQ ID NO: 1). The two bridge probes (EGFR-BP2, SEQ ID NO: 2 and EGFR-BP3, SEQ ID NO: 3) were designed to target EGFR genomic sequence. Each bridge probe comprised a targeting sequence (TS1 or TS2) region of about 25bp, a linker comprising at least 15 thymine, and a landing sequence (LS1 or LS2, italicized) having 20 bp that were designed to be complementary to the bridge binding sequence on the adaptor anchor probe. The adaptor anchor probe comprised the two bridge binding sequences (BBS1 or BBS2) that were designed to hybridize to either of the landing sequences of the bridge probes. The adaptor anchor probe further biotinylated at the 5' of the nucleic acid sequences. **FIG. 4** provides a schematic view of the synergistic indirect hybridization.

**TABLE 1. Sequence Listing**

| **SEQ ID NO.** | **ID** | **Type** | **Sequence** |
|---|---|---|---|
| 1 | UP | Adaptor anchor probe | 5’-TTTTTTTTTTGGCACCAGACTTAATCTAA GCAGAGAACATGATAAGAGA-3’ |
| 2 | EGFR-BP2 | Bridge probe | 3'- TTAGATTAAGTCTGGTGCCATTTTTTTTTTTTTTTTCA AGGAATTAAGAGAAGCAACATC-5' |
| 3 | EGFR-BP3 | Bridge probe | 3'- TCTCTTATCATGTTCTCTGCTTTTTTTTTTTTTTGAA AGCCAACAAGGAAATCCTCGAT-5' |
| 4 | EGFR Fw | EGFR forward primer | 5'-CCCGTCGCTATCAAGGAATTAAGA-3' |
| 5 | EGFR Rev | EGFR reverse primer | 5'-CCACACAGCAsAAGCAGAAACTCAC-3' |

For the hybridization capture, 20ng of fragmented (peak size 160bp) gDNA was mixed with the two bridge probes (1 fmole each) against EGFR, as well as one universal anchor probe (200 fmole) in a final solution volume of 20 ul. DNA input and hybridization probes were denatured in hybridization buffer at 95°C for 30 min, and were allowed to cool-down gradually to 65°C. The hybridization complexes were incubated at 65°C for 1 hour on a thermo cycler. The final hybridization buffer comprised 100ng/ul of blocking DNA, 1ug/ul Bovine Serum Albumin (BSA), 1ug/ul Ficoll, 1ug/ul Polyvinylpyrrolidone (PVP), 0.075M sodium citrate, 0.75 M NaCl, 5x SSC and 1X Denhardt's solutions.

To capture/clean-up, the hybridization assemblies were incubated with streptavidin beads (Thermo Fisher Dynabeads M270 Streptavidin) at room temperature for 10 min. The clean-up was conducted with three washes (wash 1: 5X SSPE, 1%SDS; wash 2: 2X SSPE, 0.1% SDS; wash 3: 0.1X SSPE, 0.01% triton).

The enriched DNA was evaluated by qPCR using primers (SEQ ID NOS. 4 & 5) against EGFR targeting sequence. The qPCR result for the captured EGFR DNA was compared to the same portion of gDNA without capture enrichment. 65% to more than 90% of EGFR was recovered.

### Example 2

### Capture by different hybridization schemes

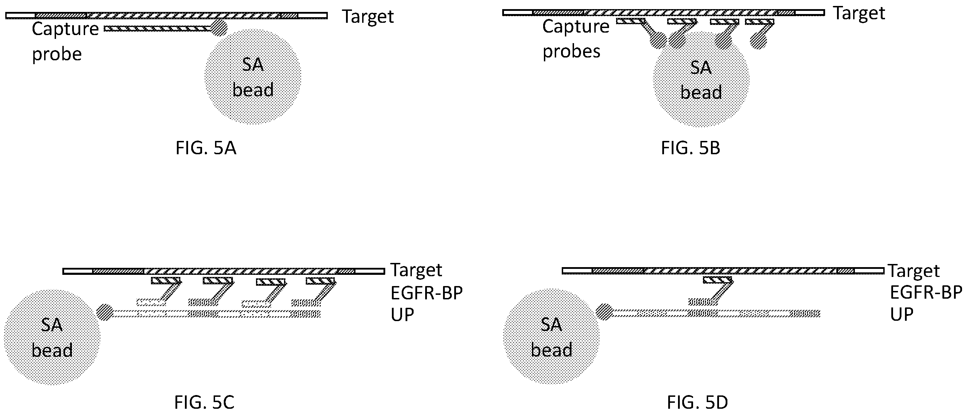

To determine the capture performance of various hybridization systems, four types of hybridization schemes were tested: non-synergistic hybridization, direct **(****FIG. 5A****),** synergistic, direct hybridization **(****FIG. 5B****),** synergistic, indirect hybridization **(****FIG. 5C****),** and non-synergistic, indirect hybridization **(****FIG. 5D****).**

The non-synergistic direct method involved hybridization of a biotinylated capture probe (120bp, SEQ ID NO. 6) comprising target specific sequence (hatched line, **FIG. 5A****).** The synergistic direct method involved hybridization of four short biotinylated capture probes (SEQ ID NOS. 7-10), and each contains 25bp of target specific sequences (hatched line, **FIG. 5B****).** The synergistic indirect method utilized four short bridge probes (SEQ ID NOS. 12-15) without biotin **(****FIG. 5C****),** and each comprised the same target specific sequences of as one of the capture probes used in the synergistic direct method. Each of the bridge probe (BP), comprised one of the two different landing sequences (dotted line and vertical hatched line) that was designed to be complementary to the one of the bridge binding sequences in the universal anchor probe (SEQ ID NO. 11). The non-synergistic but indirect method **(****FIG. 5D****)** was tested by using a short bridge probe (SEQ ID NO. 16) paired with the same universal anchor probe used in synergistic, direct hybridization. The capture probes or the universal anchor probes (UP) used in the experiements were biotinylated at the 5' ends.

**TABLE 2. Sequence Listings**

| | **SE Q ID NO.** | **ID** | **Sequence** |
|---|---|---|---|
| Non-synergistic, direct | 6 | EGFR-bio | Biotin- AGAAGGTGAGAAAGTTAAAATTCCCGTCGCTATCA AGGAATTAAGAGAAGCAACATCTCCGAAAGCCAAC AAGGAAATCCTCGATGTGAGTTTCTGCTTTGCTGTG TGGGGGTCCATGGC |
| Synergistic, direct | 7 | EGFR-bioP1 | biotin- TTTTTTTTTTGGTGAGAAAGTTAAAATTCCCGTCG |
| | 8 | EGFR-bioP2 | biotin-TTTTTTTTTTTCAAGGAATTAAGAGAAGCAACATC |
| | 9 | EGFR-bioP3 | biotin-TTTTTTTTTTGAAAGCCAACAAGGAAATCCTCGAT |
| | 10 | EGFR-bioP4 | biotin-TTTTTTTTTTAGTTTCTGCTTTGCTGTGTGGGGGT |
| Synergistic, indirect | 11 | UP | biotin- TTTTTTGGCACCAGACTTAATCTAATTTGCAGAGAA CATGATAAGAGATTTTGGCACCAGACTTAATCTAAT TTGCAGAGAACATGATAAGAGA |
| | 12 | EGFR-BP1 | TCTCTTATCATGTTCTCTGCTTTTTTTTTTTGGT GAGAAAGTTAAAATTCCCGTCG |
| | 13 | EGFR-BP2 | TTAGATTAAGTCTGGTGCCATTTTTTTTTTTTTTTCA AGGAATTAAGAGAAGCAACATC |
| | 14 | EGFR-BP3 | TCTCTTATCATGTTCTCTGCTTTTTTTTTTTTTTGAA AGCCAACAAGGAAATCCTCGAT |
| | 15 | EGFR-BP4 | TTAGATTAAGTCTGGTGCCATTTTTTTTTTTTTTAGT TTCTGCTTTGCTGTGTGGGGGT |
| Non-synergistic, indirect | 11 | UP | biotin- TTTTTTGGCACCAGACTTAATCTAATTTGCAGAGAA CATGATAAGAGATTTTGGCACCAGACTTAATCTAAT TTGCAGAGAACATGATAAGAGA |
| | 16 | EGFR-BP2 | TTAGATTAAGTCTGGTGCCATTTTTTTTTTTTTTTCA AGGAATTAAGAGAAGCAACATC |
| | 17 | EGFR Fw | CCCGTCGCTATCAAGGAATTAAGA |
| | 18 | P7 primer | CAAGCAGAAGAC GGCATACGAGAT |
| | 19 | P5 primer | AATGATACGGCGACCACCGA |

Prior to the hybridization reaction, 10ng of cfDNA was used to construct NGS library using NEBNext Ultra II DNA library prep kit by following the steps in the accompanied protocol. After the library construction, hybridization-based capture was conducted directly with the ligation mix without beads purification to enrich the library. The enriched library was then subjected to qPCR analysis.

The capture efficiency was evaluated by comparing the percentage of EGFR presence before and after capture. The ct of after capture was compared to 2.5ng of human gDNA library (the proper fraction of the capture input). The capture efficiency PCR was conducted by using primer designed against EGFR (SEQ ID NO. 17), and NGS adaptor P7 sequence (SEQ ID NO. 18). The background (total DNA presence) was evaluated by qPCR using primers (SEQ ID NOS. 18, 19) that can amplify all the DNA library. All the background delta ct was normalized to the average CT obtained from "C" probe design.

Indirect, synergistic hybridization capture demonstrated superior hybridization sensitivity and specificity over any of the non-synergistic methods and direct methods (Table 3). The synergistic indirect probe design demonstrated the highest capture efficiency (~91% on average) and lowest background noise. The non-synergistic, direct hybridization showed none to 14.87% recovery at a much higher (300x) bridge probe concentration, but showed more than 200-fold increase of background. Lowering hybridization temperature did not help on the capture efficiency, but instead dramatically increased the background noise. For the synergistic but not indirect design, neither increase of bridge probe concentration nor lowering the hybridization helped the capture efficiency. For indirect, non-synergistic method, no capture enrichment was detected.

**TABLE 3. Capture performance of various hybridization schemes.**

| **Probe conc.** | 10 fmole probes in 50ul | | 3 pmole probes in 50ul | | 3 pmol probes in 50ul | |
|---|---|---|---|---|---|---|
| | 60 ^{°}C Hybridization | | 60 ^{°}C Hybridization | | 55 ^{°}C Hybridization | |
| | **Capture Efficiency** | **Backgrou nd** | **Capture Efficienc y** | **Backgroun d** | **Capture Efficienc y** | **Backgroun d** |
| **Non-synergistic direct** | N/D | 1.4X | 14.87% | 256.0X | N/D | 128.0X |
| | 1.0% | 1.4X | 9.81% | 294.1X | 1.27% | 137.2X |
| **Synergistic direct** | N/D | 1.4X | N/D | 1.3X | N/D | 1.2X |
| | 0.6% | 1.3X | 0.70% | 1.1X | 1.03% | 1.1X |
| **Synergistic indirect** | 94.0% | 0.9X | | | | |
| | 76.3% | 1.1X | | | | |
| | 90.1% | 0.9X | | | | |
| | 84.1% | 1.1X | | | | |
| | 107.2% | 1.0X | | | | |
| | 100.0% | 1.0X | | | | |
| **Non-synergistic indirect** | 0.0% | 1.1X | | | | |
| | 0.1% | 1.1X | | | | |

### Example 3

### Indirect capture by universal anchor probe with or without spacers

A study was conducted to see if presence of spacers in-between the two or more bridge binding sequences on a universal anchor probe (UP) affect the capture performance of indirect, synergistic hybridization capture. The same bridge probes were used in both cases.

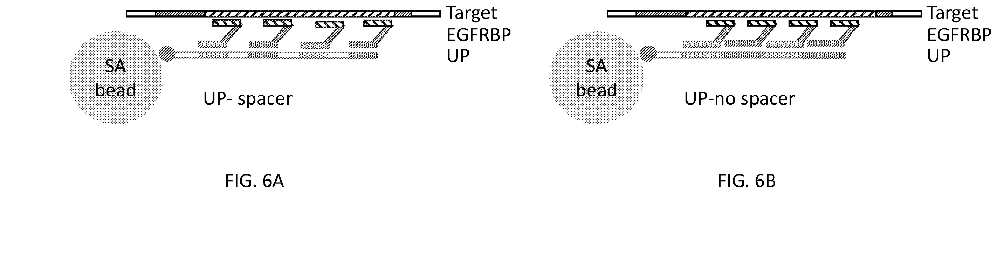

Table 4 lists the sequences of the bridge probes and UP used. **FIG. 6A** shows a schematic view of the synergistic, indirect hybridization using UP with spacer. **FIG. 6B** shows the synergistic, indirect hybridization using UP without spacer.

**TABLE 4. Sequence Listings**

| **SEQ ID NO.** | **ID** | **Spacer between landing sequences** | **Sequence** |
|---|---|---|---|
| 20 | UP-spacer | Yes | biotin- TTTTTTGGCACCAGACTTAATCTAATTTGCAGAGAACATGA TAAGAGATTTTGGCACCAGACTTAATCTAATTTGCAGAGAA CATGATAAGAGA |
| 21 | UP-no spacer | No | biotin- TTTTTTGGCACCAGACTTAATCTAAGCAGAGAACATGATAA GAGATGGCACCAGACTTAATCTAAGCAGAGAACATGATAA GAGA |
| 22 | EGFR-BP1 | TCTCTTATCATGTTCTCTGCTTTTTTTTTTTTTTGGTGAGAAAGTTAAA ATTCCCGTCG | |
| 23 | EGFR-BP2 | TTAGATTAAGTCTGGTGCCATTTTTTTTTTTTTTTCAAGGAATTAAGA GAAGCAACATC | |
| 24 | EGFR-BP3 | TCTCTTATCATGTTCTCTGCTTTTTTTTTTTTTTTTGAAAGCCAACAAGG AAATCCTCGAT | |
| 25 | EGFR-BP4 | TTAGATTAAGTCTGGTGCCATTTTTTTTTTTTTTAGTTTCTGCTTTGCT GTGTGGGGGT | |

Capture efficiency and the background noise were determined for either hybridization capture. The background noise was calculated by normalizing the qPCR result to the average background signal. The capture efficiency was not largely influenced by the presence of spacer, but the background noise of the capture hybridization without spacers was about 100-fold higher than the capture with spacer (Table 5). Hence, it suggests that the spacers in universal anchor probe played a significant role in enabling a highly specific (low background) capture.

**TABLE 5. Capture performance of hybridization with universal anchor probes with or without spacers**

| | **Capture Efficiency** | **Background** |
|---|---|---|
| **UP-spacer** | 75.8% | 1.1X |
| | 70.7% | 0.9X |
| **UP-no spacer** | 66.0% | 93.7X |
| | 66.0% | 107.6X |

### Example 4

### Determination of NGS metric using synergistic indirect capture method

The next generation sequencing (NGS) metric using 3, 15, and 76 target panel were determined. The mapped rate was calculated as the percentage of sequencing read that was aligned to the human genome. The mapped rates for 3, 15, and 76 target panel were 97%, 94%, 95%, respectively (Table 6). The on-target rates were calculated using deduped mapped read over the region covered by capture probe and 100bp flanking. For the small panel such as 3, 15 and 76-targets, conventional hybridization-based DNA enrichment was not feasible. However, the study showed comparably high on-target rate of 83.6% and 85.3% for the 15 and 76-target panel compared to standard target panel with more than 50kb.

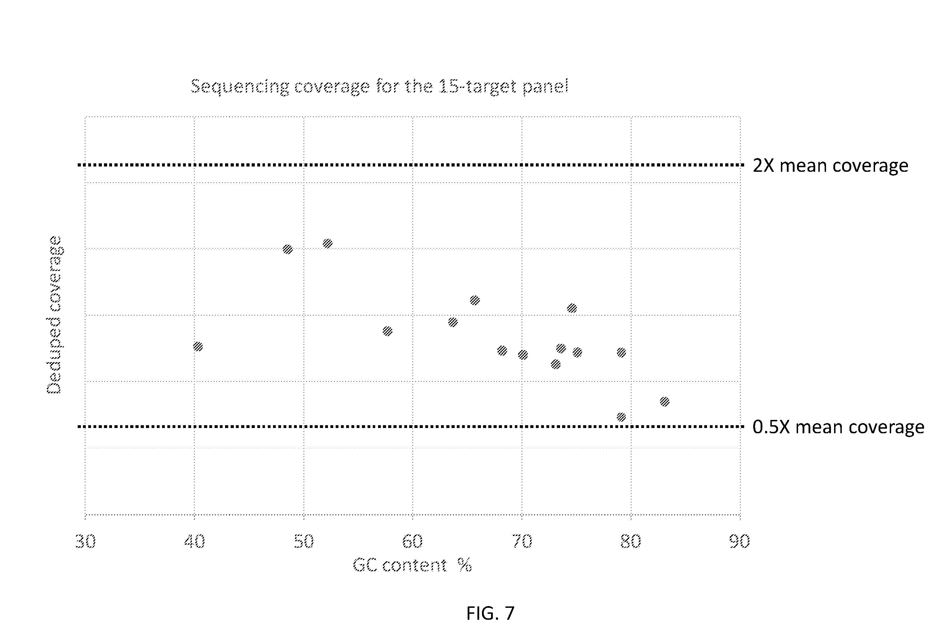

Moreover, the uniformity for the panels were high (>99% of the position had reads higher than 0.2x of the mean coverage, and more than 95% for 0.5x coverage). 0.2 or 0.5X coverage was not suitable for the micro-panel with 3 targets. The high uniformity the 15-target panels was also reflected by the even coverage at the regions where the GC content is high **(****FIG. 7****).** The coverage of the region at 80% GC content was higher than 0.5x of the mean coverage.

**TABLE 6. NGS metric using synergistic indirect capture method**

| | **3-target (n=3)** | **15-target (n=5)** | **76-target (n=6)** |
|---|---|---|---|
| **Mapped rate** | 97.0% | 93.8% | 95.7% |
| **On-target rate** | 14.3% | 83.6% | 85.3% |
| **0.2X coverage** | NA | 98.6% | 99.2% |
| **0.5X coverage** | NA | 88.6% | 98.2% |

### Example 5

### Determination of NGS metric of human SNPs using synergistic indirect capture method

A synergistic indirect hybridization assay was conducted to cover 76 human ID single-nucleotide polymorphisms (SNPs). A pre-amplification hybridization was conducted on 20 ng of human cell-free DNA (cfDNA). The result was compared to that of the post-amplification hybridization using the commercially available IDT xGen Hybridization and Wash Kit. xGen Human ID Research Panel V1.0 covering the same 76 ID SNPs was used for the capture. The xGEN human ID panel was used to conduct hybridization-based capture on the NGS library constructed using 20ng of cfDNA as original input by following the commercial protocol.

The next generation sequencing (NGS) metric using the 76-target panel was determined (Table 7). The target rate of the post-amplification capture was low at 30.7% on target rate. In contrast, the on-target rate of the SICON-MAS panel covering the same genomic region was 88%.

**TABLE 7. NGS metric using synergistic indirect capture method**

| **Capture** | **SICON-MAS pre-amp** | **IDT xGEN post-amp** |
|---|---|---|
| **Mapped rate** | 99.5% | 97.7% |
| **On-target rate** | 88% | 30.7% |
| **0.2X coverage** | 100% | 100.0% |
| **0.5X coverage** | 96% | 94% |

### Example 6

### Comparison of SICON-SEQ with post-amplification method

Synergistic indirect capture of nucleic acid for sequencing (SICON-SEQ) was conducted for a panel of 76 human gene targets provided >80% on-target rate for 1M reads from 10 ng cfDNA input, with only 1 hour of pre-amplification capture. Post-amplification capture with company "I" kit was used for the same panel to only yield 6-30% on target rate for 1M read from double amount of input (20ng cfDNA) with 16 hours of post amplification capture. A pre-amplification capture using the company I kit conducted but failed to generate any results.

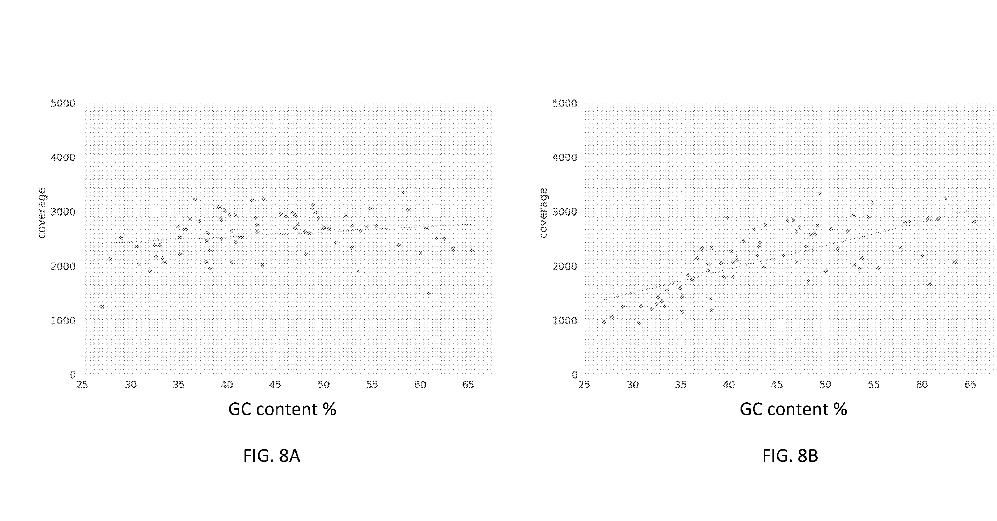

**FIGS. 8A-8B** show the coverage by SICON-SEQ and IDT xGen Hybridization and Wash Kit over areas of different percentage of GC contents. The coverage from regions with low GC content (<30%) to high GC content (>50%) were very uniform for SICON-SEQ assay **(****FIG. 8A****).** For the capture protocol using IDT xGEN kit **(****FIG. 8B****)** that yielded no library enrichment, the coverage of regions with different CG content was systematically biased.

### Example 7

### Methylation assay by SICON-TMS

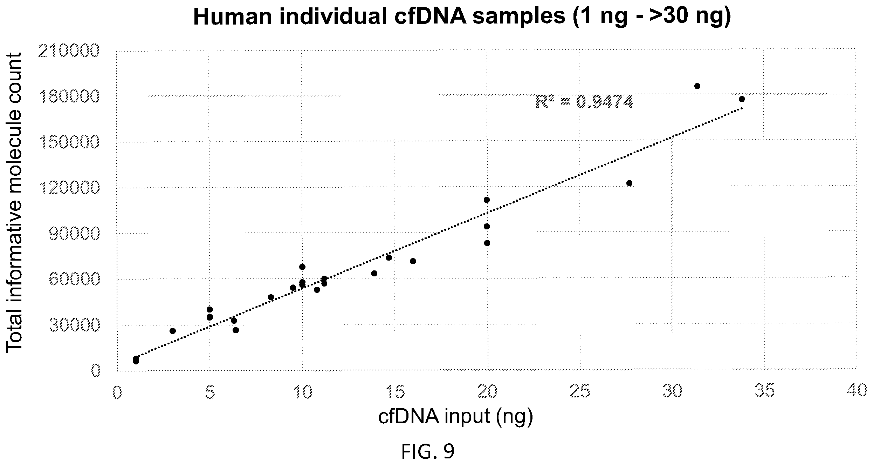

A SICON targeted methylation sequencing (SICON-TMS) assay was conducted as illustrated in FIGS. 2A and 2B. The sample cfDNA were extracted from 3-5 ml of plasma from difference non-cancerous individuals and interrogated for 120 different differential methylated regions (DMRs). The read-out showed near linear (R²=0.9474) relationship to the input, even as low as 1ng of cfDNA input **(****FIG. 9****).**

### Example 8

### Detection of methylated DNA in cfDNA by SICON-TMS

A SICON-TMS assay was conducted to interrogate 60 different differential methylated regions (DMRs).

A new-generation sequencing (NGS) library was first constructed using cfDNA by following NEBNext Ultra II kit manual. The library DNA (cfDNA with spike in methylated DNA at ratio of 0.01%, 0.1%, 1%, 10%, or 100%) was inputted for hybridization capture. 20 ng of DNA without amplification was mixed with probes and the library/probe mixtures were denatured in hybridization buffer at 95°C for 30 min. The mixture was allowed to gradually cool down to 60°C. The hybridization mixtures were incubated at 60°C for 1 hour on a thermo cycler. The final hybridization buffer contained 100 ng/ul of salmon sperm DNA, 1 ug/ul Bovine Serum Albumin (BSA), 1 ug/ul Ficoll, 1 ug/ul polyvinylpyrrolidone (PVP), 0.075M sodium citrate, 0.75 M NaCl, 5x SSC and 1X Denhardt's solutions.

For the clean-up, the captured assembly was incubated with streptavidin beads (Thermo Fisher Dynabeads M270 Streptavidin) at room temperature for 10 min and followed by three washes (wash 1 :5X SSPE, 1%SDS; wash 2: 2X SSPE, 0.1%; wash 3: 0.1X SSPE, 0.01% triton). The cleaned-up assembly was treated with bisulfite for methylation analysis.

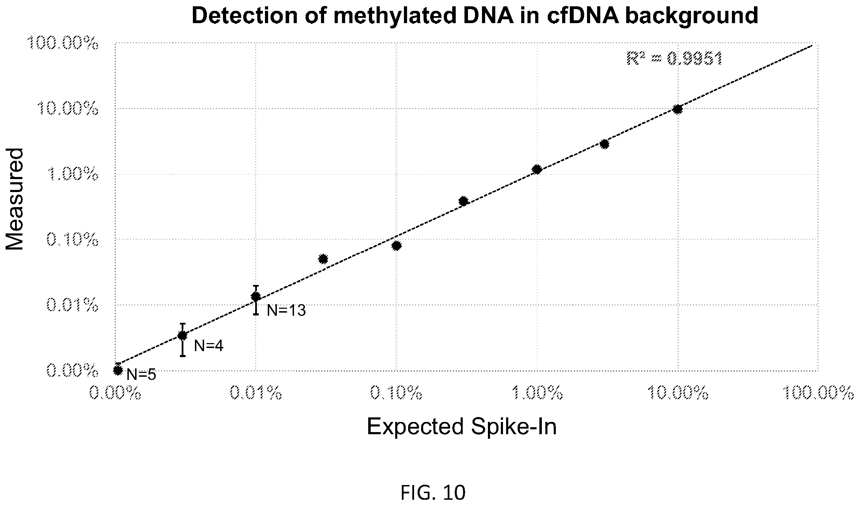

**FIG. 10** shows the relationship between the expected spike-in and the measured value. SICON-TMS assay demonstrated analytical sensitivity and linearity down to 0.01% methylation. The methylation percentage highly correlated with the expected value, with a R² of 0.99, indicating the high accuracy of the assay.

### Example 9

### Detection of cancer methylation pattern in cfDNA by SICON-TMS

Samples from normal colon tissue and colon cancer tissue, as well as samples of plasma cfDNA from a healthy individual and a colon cancer patient were bisulfite treated and sequenced. Sequencing reads were mapped to each differentially methylated region (DMR) are de-duplicated. Each of the resulting reads contained the CpG methylation information from a unique DNA molecule captured by the assay. Two metrics were then calculated for each read:
1) N: the total number of CpGs in the read;
2) M: the number of methylated CpGs in the read.
From 1) and 2), a third metric was calculated as:
3) f = M/N, the fraction of CpGs that are methylated in the current read.

The results are shown as scatter plots showing f (y axis) vs N (x axis) for each DMR, with every read in the DMR shown as a dot in the plot. FIG. 11 shows the molecule methylation scatter pattern of DMR1 in the normal colon tissue (FIG. 11A) and the colon cancer tissue genomic DNA (FIG. 11B). It demonstrates a DMR where there is no hyper-methylated DNA molecule in normal colon tissue and a large amount of hyper-methylated molecules in colon cancer tissue.

FIGS. 12A and 12B show the molecule methylation scatter pattern of DMR2 in the normal colon tissue and the colon cancer tissue genomic DNA respectively. These figures demonstrate a DMR where there are some hyper-methylated DNA molecules in normal colon tissue and a larger amount of hyper-methylated molecules in colon cancer tissue.

FIGS. 13A and 13B show the molecule methylation scatter pattern of DMR1 and DMR2 in health individual's plasma cfDNA and a colon cancer patient's plasma cfDNA respectively. The counts of hyper-methylated molecules illustrated in the upper part of FIG. 13B from each DMR may be used as the basis for disease detection from liquid biopsy.

### Example 10

### Detection of cancer methylation pattern in cfDNA by SICON-TMS

A Point-n Seq colorectal cancer (CRC) panel covering 100 methylation markers was designed in 3 steps. First, approximately 1000 CRC-specific markers were identified from public databases. Secondly, makers with high background signal in baseline cfDNA of healthy population were eliminated. Finally, the list was finalized to contain the most differentiating markers between patient and healthy cfDNA. The capture of the SICON CRC panel was highly efficient resulting in high uniformity (94% > 0.5X, 100% >0.2X) and on-target rate (>80%). For 20ng cfDNA input, more than 1000 deduped informative reads were obtained for each marker on average, despite the high GC content (> 80%). The output of informative reads was linear to the cfDNA input ranging from 1ng to 40ng. In titration studies, 0.6pg (0.2X genome equivalent) methylated DNA in 20ng cfDNA (0.003%) was reliably detected over cfDNA background. In a pilot clinical study using plasma samples from patients with colorectal adenocarcinoma - early stage (I, n=7; II, n=7), late stage (III, n=11; IV, n=3), and control individuals (n=105), the average fractions of methylated signal were 0.0034%, 0.013%, 0.09%, 0.17%, 0.29% for control, stage I, II, III, IV accordingly. The methylation fraction of stage I samples was significantly different from the control group (P<0.001). With a simple cut-off using methylation fraction, the Point-n Seq CRC panel achieved a sensitivity of 86% for stage I, 100% for stage (II-IV) at a specificity of 91%, with AUC=0.96.

### Example 11

### Point-n-Seq SNV + Methyl dual capture analysis on CRC plasma samples

Genetic and epigenetic alternations were detected by unified Point-n-Seq assay in plasma samples (1ml) from late stage CRC patients. A Point-n-Seq colorectal cancer (CRC) panel was designed covering methylation markers and >350 hotspot mutations from 22 genes.

Two sequential rounds of target enrichment were performed by synergistic, indirect hybridization capture as described herein using the methylation marker panel and the mutation hotspot panel. Briefly, 20µL of each cfDNA sample was added into a PCR tube. For DNA volumes less than 20µL, IDTE or Buffer EB was added to a final volume of 20µL. For each sample 2.8 µL of end prep buffer and 1.2 µL of end prep enzyme were added. The tubes were mixed well by gentle vortexing, then briefly centrifuged. The tubes were run in a thermal cycler with a heated lid at a temperature of 20°C for 30 min followed by 65°C for 30 min. Following this 2.5 µL of the adapter solution was added, and 13 µL of ligation mix and the mix was incubated at 20°C for 30 min.

The Sample binding beads were equilibrated to room temperature for at least 15 minutes, and vortexed to resuspend. 48 µL (~1.2x volume) of Library Binding Beads was added to the 39.5µL Ligation reaction. These were mixed thoroughly by pipetting at least 10 times and briefly centrifuged. The mix was incubated for 10 min at room temperature and placed on a magnet for at least 2 min or until the solution is clear. The supernatant was removed and discarded. On magnet, 150 µL of Sample Wash Buffer was added to beads without disturbing the beads, incubated for 2 min, and supernatant was discarded.

For target capture a hybridization mix containing the mutation capture panel and probe binding mix was added and mixed well by gentle vortexing or flicking. The mixture was heated to 98 °C for 2 min, and then ramped down to 60 °C at a rate of 2.5 °C /s, and incubated at 60°C for 60 min. After the 60 min hybridization the samples were placed on a magnet for 30 sec and the supernatant was carefully transferred to labeled tubes, and saved for the second hybridization step. The beads were washed 3 times and resuspended, and the DNA was amplified on the bead.

The saved supernatant from above was mixed with hybridization mix containing the TMS capture panel, and capture hybridization was performed as for the mutation capture panel. The captured TMS DNA was bisulfide treated, repaired, and eluted from the beads followed by index PCR. Both amplified DNA samples were prepared for sequencing and sequenced on the Illumina platform.

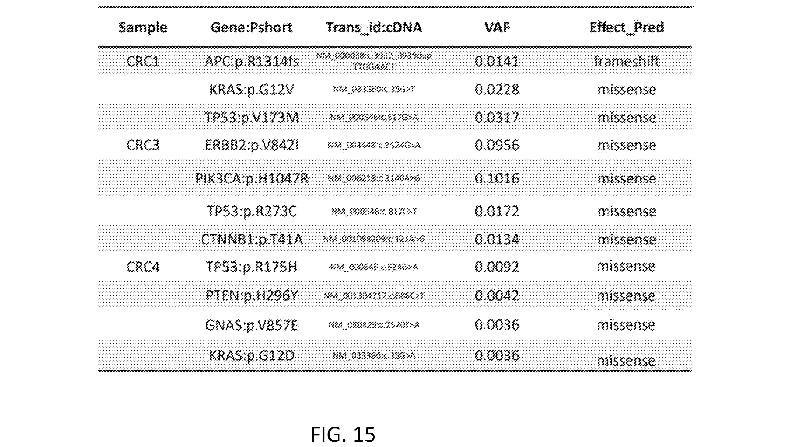

Figure 14 illustrates the sequential target enrichment. Table 8 lists the DNA input amounts, and the fractions of methylated signal and the fraction of mutant signal for each patient sample. Details of the detected mutations are shown in FIG. 15. As shown by Table 8 the capture of the Point-n-Seq CRC mutation and methylation panels was highly efficient resulting in detection of hypermethylation and mutations from a wide range of starting quantities of DNA. Furthermore, the methylation and mutation combined analysis using plasma cfDNA from CRC patients showed consistent tumor content estimation from methylation status and driver mutation allele frequency.

**Table 8**

| | Plasma volume (ml) | DNA input (ng) | Methylated signal % | Mutation % |
|---|---|---|---|---|
| CRC 1 | 1 | 2.74 | 1.09% | 4.40% |
| CRC 2 | 1 | 6 | 0.34% | 0.00% |
| CRC 3 | 1 | 6 | 5.12% | 11.00% |
| CRC 4 | 1 | 4.95 | 0.19% | 1.80% |
| CRC 5 | 1 | 49.8 | 4.38% | 0.00% |

### Example 12

### The methylation signal from dual analysis is comparable with stand alone methylation (TMS) analysis

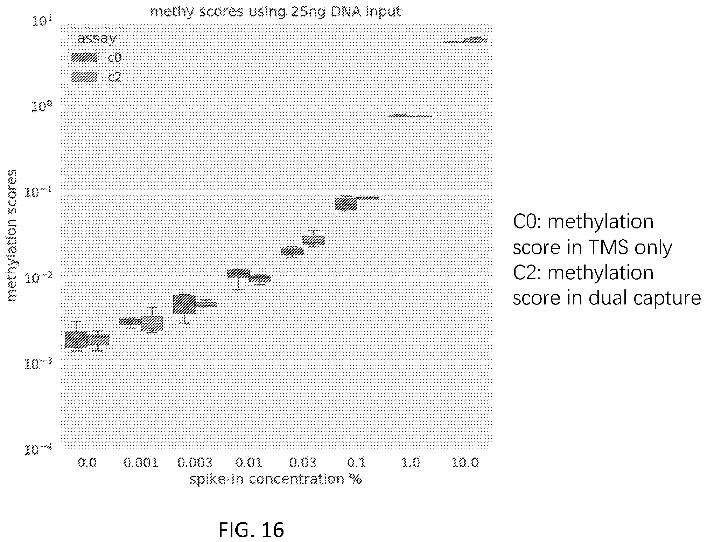

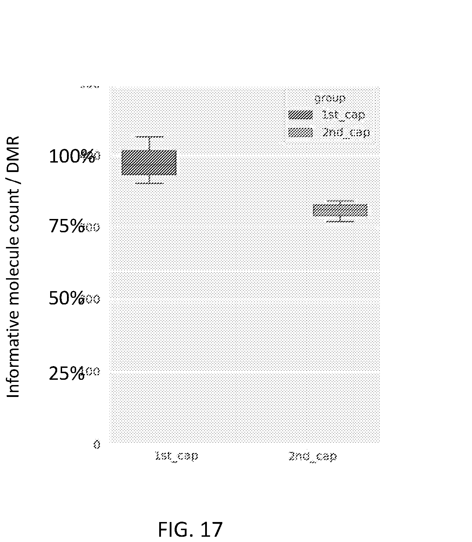

To assess the methylation signal derived from the sequential target enrichment method a titration experiment was performed with gDNA from cell line HCT116 spiked into control cfDNA. The HCT116 gDNA was spiked at concentrations ranging from 0.001% to 10%. The same DNA input was subjected to TMS analysis alone or mutation-TMS dual analysis by sequential SICON, where the enrichment step for the mutation analysis was performed first and the enrichment step for the TMS analysis was performed second as outlined in FIG. 14. As shown in FIG.16 the methylation scores from the stand alone and dual analysis were comparable indicating the methylation assay sensitivity was not compromised as the second capture in the sequential capture dual analysis. FIG. 17 shows that the 2^{nd} capture TMS recovery (informative molecule count from the sequencing per differentially methylated region (DMR)) is about 85% of the 1^{st} capture TMS.

### Example 13

### Tumor-informed personalized panel analysis

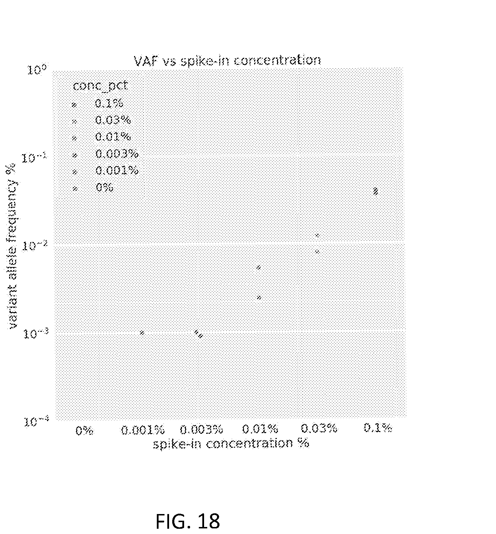

CRC tumor gDNA was subjected to whole exon sequencing and 114 single nucleotide variants were selected to make a personalized panel. The CRC tumor gDNA was spiked into control cfDNA in a titration experiment at concentrations of 0.001%, 0.003%, 0.01, 0.03%, and 0.1%. As shown in FIG. 18 the sample spiked at 0.003% could be separated from 0% suggesting a limit of detection of 0.003% for the particular personalized hybridization-based assay. It is expected that a larger panel would result in a lower detection limit.

## Claims

1. A method comprising:
obtaining a template nucleic acid molecule attached to a 5' adaptor or a 3' adaptor;
hybridizing a first target specific region of a first bridge probe to a first target sequence of the template nucleic acid molecule, wherein a first adaptor landing sequence of the first bridge probe is configured to hybridize to a first bridge binding sequence of an adaptor anchor probe; and
hybridizing a second target specific region of a second bridge probe to a second target sequence of the template nucleic acid molecule, wherein a second adaptor landing sequence of the second bridge probe is configured to hybridize to a second bridge binding sequence of the adaptor anchor probe,
wherein the first bridge probe and second bridge probe each comprises DNA or RNA.

2. The method of claim 1,
(i) wherein the first bridge probe and/or second bridge probe comprises uracil;
(ii) wherein the first bridge probe and/or second bridge probe comprises methylated cytosine;
(iii) wherein the first bridge probe and/or second bridge probe comprises one or more molecular barcodes;
(iv) wherein the first bridge probe and/or second bridge probe comprises one or more binding moieties;
(v) wherein the first bridge probe and/or second bridge probe comprises a label,
preferably wherein the label is a fluorescent label, radioactive label, or biotin;
and/or
(vi) wherein the first bridge probe and/or second bridge probe comprises a linker that comprises a non-nucleic acid polymer.

3. The method of claim 1 or 2,
(i) wherein the template nucleic acid molecule is genomic DNA, mitochondrial DNA, viral DNA, cDNA, or synthetic DNA;
(ii) wherein the template nucleic acid molecule is double-stranded DNA, single-stranded DNA, fragmented DNA, or damaged DNA;
(iii) wherein the template nucleic acid molecule is cell-free DNA,
optionally:
(a) wherein the cell-free DNA is from a fetal source;
or
(b) wherein the cell-free DNA is circulating tumor DNA;
(iv) wherein the template nucleic acid molecule is mRNA, tRNA, microRNA, snRNA, piRNA, small non-coding RNA, polysomal RNA, intron RNA, pre-mRNA, viral RNA, or cell-free RNA;
(v) wherein the template nucleic acid molecule is a modified nucleic acid comprising one or more heterocyclic bases,
optionally wherein the one or more heterocyclic bases are selected from a group consisting of methylated purines or pyrimidines, acylated purines or pyrimidines, and alkylated riboses;
(vi) wherein the template nucleic acid molecule is a modified nucleic acid comprising one or more modified sugar moieties,
optionally wherein the one or more modified sugar moieties are selected from a group consisting of hexitol nucleic acid, peptide nucleic acid, locked nucleic acid, threose nucleic acid, and glycerol nucleic acid;
and/or
(vii) wherein the template nucleic acid is a damaged nucleic acid,
optionally:
(a) wherein the damaged nucleic acid comprises altered or missing bases and/or a modified backbone;
and/or
(b) wherein the damaged nucleic acid has been damaged by oxidation, radiation, random mutation, or bisulfite treatment.

4. The method of any one of claims 1-3, further comprising treating the template nucleic acid molecule with a methylation assay reagent after the hybridizing of the first target specific region and the hybridizing of the second target specific region,
preferably wherein the methylation assay reagent is bisulfite or an enzyme that modifies methylated cytosine.

5. The method of claim 4, further comprising sequencing of the template nucleic acid molecules and analysing of the methylation sequencing data in a method comprising the following steps:
counting the total number (N) of CpG sites of each sequenced molecule;
counting the number (M) of methylated CpG sites of each sequenced molecule;
calculating for each sequenced molecule the fraction (f) of methylated CpG sites by dividing the number of methylated CpG sites (M) by the total number of CpG sites (N); and
applying a downstream classification model, wherein each data pair (N, f) represents one data point in the downstream classification model.

6. The method of claim 5,
(i) further comprising applying a hyper-methylation filter threshold (f0), wherein each sequenced molecule with a methylation fraction above the hyper-methylation filter threshold (f0) is counted as a hyper-methylated molecule,
optionally:
(a) wherein the hyper-methylation filter threshold (f0) is set based on the analysis of methylation in normal tissue or a sample from a healthy subject;
(b) wherein the hyper-methylation filter threshold (f0) is set at 0.2, 0.3, 0.4, 0.5. 0.6, 0.7, 0.8, or 0.9;
or
(c) wherein the hyper-methylation filter threshold (f0) is set as 0.5, 1, 1.5, 2, 2.5, or 3 standard deviations from the mean methylation fraction in a normal tissue sample or a sample from a healthy subject;
and/or
(ii) further comprising applying an additional threshold NO to only count reads with N>N0.

7. A method for sequential hybridization-based target enrichment comprising:
obtaining a sample comprising a plurality of nucleic acid molecules;
performing a first target enrichment to enrich for nucleic acid molecules comprising sequences corresponding to a first panel of one or more genome regions, thereby generating a first enriched sample comprising nucleic acids enriched for sequences corresponding to the first panel of one or more genome regions and a remaining sample comprising nucleic acids depleted for sequences corresponding to the first panel of one or more genome regions;
performing a second target enrichment upon the remaining sample to enrich for nucleic acid molecules comprising sequences corresponding to a second panel of one or more genome regions, thereby generating a second enriched sample comprising nucleic acids enriched for sequences corresponding to the second panel of one or more genome regions; wherein the first panel of one or more genome regions and the second panel of one or more genome regions are different; and
performing a first analysis of the first enriched sample and a second analysis of the second enriched sample;
wherein the first target enrichment and second target enrichment each comprise:
hybridizing a first target specific region of a first bridge probe to a first target sequence of a molecule with a sequence corresponding to the genome region, wherein a first adaptor landing sequence of the first bridge probe is bound to a first bridge binding sequence of an adaptor anchor probe; and
hybridizing a second target specific region of a second bridge probe to a second target sequence of the molecule with a sequence corresponding to the genome region, wherein a second adaptor landing sequence of the second bridge probe is bound to a second bridge binding sequence of the adaptor anchor probe.

8. The method of claim 7,
(i) wherein the first analysis is a sequence analysis and the second analysis is methylation analysis;
(ii) wherein the first analysis is a first sequence analysis, and the second analysis is a second sequence analysis, wherein the first sequence analysis is performed at a different depth of sequencing than the second sequence analysis;
(iii) wherein the adaptor anchor probe comprises a binding moiety and the method further comprises attaching the binding moiety to a support and separating the support with the attached binding moiety from the unbound nucleic acids;
(iv) wherein the first or second panel of one or more genome regions comprises a panel of genome regions associated with differentially methylated regions, with hypermethylation in cancer, with epigenetic modifications, with introns, with promoters, or with other regulatory sequences,
preferably wherein the first panel of one or more genome regions comprises a panel of genome regions associated with mutation hotspots and wherein the second panel of one or more genome regions comprises a panel of genome regions associated with methylated regions;
(v) wherein the first or second panel of one or more genome regions comprises a panel of genome regions associated with mutation hotspots, oncogenes, tumor suppressor genes, oncogene exons, tumor suppressor exons, or regulatory regions;
(vi) wherein the first enriched sample is analysed by sequencing to asses mutations while the second enriched sample is bisulfite treated or enzymatically treated prior to sequencing to assess methylation;
(vii) wherein the analysis of the first enriched sample is performed prior to performing a second target enrichment step,
preferably wherein the analysis of the first enriched sample is used to select a second panel for the second enrichment step;
and/or
(viii) wherein the method further comprises performing third, fourth, or further rounds of target enrichment with third, fourth, or further panels of genome regions.

9. The method of claim 8,
(i) wherein the method further comprises attaching adaptors to the 5' ends or the 3' ends of nucleic acid molecules of the plurality of nucleic acid molecules;
and/or
(ii) wherein the nucleic acid molecules are DNA, genomic DNA, mitochondrial DNA, viral DNA, cDNA, cell-free DNA, synthetic DNA, mRNA, tRNA, microRNA, snRNA, piRNA, small non-coding RNA, polysomal RNA, intron RNA, pre-mRNA, viral RNA, or cell-free RNA.

10. A nucleic acid complex comprising a template nucleic acid molecule,
wherein a 5' end or a 3' end of the template nucleic acid molecule is attached to an adaptor;
wherein a first target sequence of the template nucleic acid molecule is hybridized to a first target specific region of a first bridge probe and a second target sequence of the template nucleic acid molecule is hybridized to a second target specific region of a second bridge probe; and
wherein a first adaptor landing sequence of the first bridge probe is bound to a first bridge binding sequence of an adaptor anchor probe and a second adaptor landing sequence of the second bridge probe is bound to a second bridge binding sequence of the adaptor anchor probe.

11. A nucleic acid complex according to claim 10 for use in a method of diagnosis or detection of a disease or condition.

12. The nucleic acid complex for use according to claim 11, wherein the disease or condition is connected to methylation abnormalities,
preferably wherein the method comprises generating the methylation pattern of cell-free DNA as a marker of cancer.

13. The nucleic acid complex for use according to claim 11 or 12, wherein the disease or condition is selected from:
(i) a cancer,
preferably wherein the cancer is selected from a group consisting of colon cancer, breast cancer, liver cancer, bladder cancer, Wilms cancer, ovarian cancer, esophageal cancer, prostate cancer, bone cancer, hepatocellular carcinoma, glioblastoma, squamous cell lung cancer, thyroid carcinoma, or leukemia;
(ii) a neurological disease selected from a group consisting of Alzheimer's disease, autism spectrum disorder, Rett Syndrome, and schizophrenia;
(iii) an immunodeficiency;
(iv) a skin disease;
(v) an autoimmune disease selected from the group consisting of Ocular Behcet's disease, systemic lupus erythematosus, rheumatoid arthritis, and multiple sclerosis;
(vi) an infection;
(vii) a metabolic disease; or
(viii) a condition selected from Beckwith-Wiedemann Syndrome, Prader-Willi syndrome, or Angelman syndrome.

14. The nucleic acid complex for use according to any one of claims 11-13,
(i) wherein the method comprises combined targeted methylation sequencing and mutation analysis from cell-free DNA for cancer early detection and management;
(ii) wherein the method comprises the detection of tissue-specific methylation changes in cancer genomes for detecting circulating tumor DNA in plasma from early-stage or recurrent cancer patients;
(iii) wherein the method comprises the detection of the origin of a tumor with tissue-specific miRNA and DNA methylation markers;
(iv) wherein the method comprises determining methylation levels or fractional contributions of different tissues to a cell-free DNA mixture;
and/or
(v) wherein the method comprises the detection of fetal DNA or fetal abnormalities using a maternal sample.

## Patentansprüche

1. Verfahren, umfassend:
Beziehen eines an einen 5'-Adapter oder einen 3'-Adapter angebrachten Template-Nukleinsäuremoleküls;
Hybridisieren einer ersten zielspezifischen Region einer ersten Brückensonde an eine erste Zielsequenz des Template-Nukleinsäuremoleküls, wobei eine erste Adaptor-Andocksequenz der ersten Brückensonde dazu eingerichtet ist, an eine erste Brückenbindungssequenz einer Adaptor-Ankersonde hybridisiert zu werden; und
Hybridisieren einer zweiten zielspezifischen Region einer zweiten Brückensonde mit einer zweiten Zielsequenz des Template-Nukleinsäuremoleküls, wobei eine zweite Adaptor-Andocksequenz der zweiten Brückensonde dazu eingerichtet ist, an eine zweite Brückenbindungssequenz der Adaptor-Ankersonde hybridisiert,
wobei die erste Brückensonde und die zweite Brückensonde jeweils DNA oder RNA umfassen.

2. Verfahren nach Anspruch 1,
(i) bei dem die erste Brückensonde und/oder die zweite Brückensonde Uracil umfasst;
(ii) die erste Brückensonde und/oder die zweite Brückensonde methyliertes Cytosin umfasst;
(iii) die erste Brückensonde und/oder die zweite Brückensonde einen oder mehrere molekulare Barcodes umfasst;
(iv) die erste Brückensonde und/oder die zweite Brückensonde eine oder mehrere Bindungsgruppen umfasst;
(v) die erste Brückensonde und/oder die zweite Brückensonde eine Markierung umfasst, wobei die Markierung vorzugsweise eine Fluoreszenzmarkierung, eine radioaktive Markierung oder Biotin ist;
und/oder
(vi) die erste Brückensonde und/oder die zweite Brückensonde einen Linker umfasst, der ein Nicht-Nukleinsäure-Polymer umfasst.

3. Verfahren nach Anspruch 1 oder 2,
(i) bei dem das Template-Nukleinsäuremolekül genomische DNA, mitochondriale DNA, virale DNA, cDNA oder synthetische DNA ist;
(ii) das Template-Nukleinsäuremolekül doppelsträngige DNA, einzelsträngige DNA, fragmentierte DNA oder beschädigte DNA ist;
(iii) das Template-Nukleinsäuremolekül zellfreie DNA ist,
wobei wahlweise:
(a) die zellfreie DNA aus einer fetalen Quelle stammt;
oder
(b) die zellfreie DNA zirkulierende Tumor-DNA ist;
(iv) das Template-Nukleinsäuremolekül mRNA, tRNA, microRNA, snRNA, piRNA, kleine nicht-kodierende RNA, polysomale RNA, Intron-RNA, prä-mRNA, virale RNA oder zellfreie RNA ist;
(v) das Template-Nukleinsäuremolekül eine modifizierte Nukleinsäure ist, die eine oder mehrere heterocyclische Basen umfasst,
wobei wahlweise die eine oder mehrere heterocyclischen Basen aus einer Gruppe ausgewählt sind, die aus methylierten Purinen oder Pyrimidinen, acylierten Purinen oder Pyrimidinen und alkylierten Ribosen besteht;
(vi) das Template-Nukleinsäuremolekül eine modifizierte Nukleinsäure ist, die eine oder mehrere modifizierte Zuckereinheiten umfasst,
wobei wahlweise die eine oder mehrere modifizierten Zuckereinheiten aus einer Gruppe ausgewählt sind, die aus Hexitol-Nukleinsäure, Peptidnukleinsäure, Locked Nukleinsäure, Threose-Nukleinsäure und Glycerin-Nukleinsäure besteht;
und/oder
(vii) die Template-Nukleinsäure eine beschädigte Nukleinsäure ist,
wobei wahlweise:
(a) die beschädigte Nukleinsäure veränderte oder fehlende Basen und/oder ein modifiziertes Rückgrat hat;
und/oder
(b) die beschädigte Nukleinsäure durch Oxidation, Bestrahlung, zufällige Mutation oder Bisulfitbehandlung beschädigt wurde.

4. Verfahren nach einem der Ansprüche 1 bis 3, weiterhin umfassend die Behandlung des Template-Nukleinsäuremoleküls mit einem Methylierungs-Assay-Reagenz nach der Hybridisierung der ersten zielspezifischen Region und der Hybridisierung der zweiten zielspezifischen Region,
wobei das Methylierungs-Assay-Reagenz vorzugsweise Bisulfit oder ein Enzym ist, das methyliertes Cytosin modifiziert.

5. Verfahren nach Anspruch 4, weiterhin umfassend die Sequenzierung der Template-Nukleinsäuremoleküle und die Analyse der Methylierungssequenzierungsdaten in einem Verfahren, das die folgenden Schritte umfasst:
Zählen der Gesamtzahl (N) der CpG-Stellen jedes sequenzierten Moleküls;
Zählen der Anzahl (M) der methylierten CpG-Stellen jedes sequenzierten Moleküls;
Berechnen des Anteils (f) methylierter CpG-Stellen für jedes sequenzierte Molekül durch Dividieren der Anzahl der methylierten CpG-Stellen (M) durch die Gesamtzahl der CpG-Stellen (N); und
Anwenden eines nachgelagerten Klassifikationsmodells, wobei jedes Datenpaar (N, f) einen Datenpunkt in dem nachgelagerten Klassifikationsmodell darstellt.

6. Verfahren nach Anspruch 5,
(i) weiterhin umfassend das Anwenden eines Hypermethylierungsfilter-Schwellenwerts (f0), wobei jedes sequenzierte Molekül mit einem Methylierungsanteil oberhalb des Hypermethylierungsfilter-Schwellenwerts (f0) als hypermethyliertes Molekül gezählt wird,
wobei wahlweise:
(a) der Hypermethylierungsfilter-Schwellenwert (f0) auf der Grundlage der Analyse der Methylierung in normalem Gewebe oder einer Probe von einer gesunden Person festgelegt wird;
(b) der Hypermethylierungsfilter-Schwellenwert (f0) auf 0,2, 0,3, 0,4, 0,5, 0,6, 0,7, 0,8 oder 0,9 festgelegt wird;
oder
(c) der Hypermethylierungsfilter-Schwellenwert (f0) auf 0,5, 1, 1,5, 2, 2,5 oder 3 Standardabweichungen von dem mittleren Methylierungsanteil in einer normalen Gewebeprobe oder einer Probe von einer gesunden Person festgelegt wird;
und/oder
(ii) weiterhin umfassend das Anwenden eines zusätzlichen Schwellenwerts N0, um nur Reads mit N > N0 zu zählen.

7. Verfahren zur sequenziellen, auf Hybridisierung basierenden Zielanreicherung, umfassend:
Beziehen einer Probe, die eine Vielzahl von Nukleinsäuremolekülen umfasst;
Durchführung einer ersten Zielanreicherung zur Anreicherung von Nukleinsäuremolekülen, die Sequenzen umfassen, die einem ersten Panel aus einer oder mehreren Genomregionen entsprechen, wodurch eine erste angereicherte Probe erzeugt wird, die Nukleinsäuren enthält, die hinsichtlich Sequenzen angereichert sind, die dem ersten Panel aus einer oder mehreren Genomregionen entsprechen, sowie eine verbleibende Probe, die Nukleinsäuren enthält, die hinsichtlich Sequenzen verarmt sind, die dem ersten Panel aus einer oder mehreren Genomregionen entsprechen;
Durchführen einer zweiten Zielanreicherung an der verbleibenden Probe, um Nukleinsäuremoleküle anzureichern, die Sequenzen umfassen, die einem zweiten Panel aus einer oder mehreren Genomregionen entsprechen, wodurch eine zweite angereicherte Probe erzeugt wird, die Nukleinsäuren enthält, die hinsichtlich Sequenzen angereichert sind, die dem zweiten Panel aus einer oder mehreren Genomregionen entsprechen; wobei sich das erste Panel aus einer oder mehreren Genomregionen und das zweite Panel aus einer oder mehreren Genomregionen voneinander unterscheiden; und
Durchführen einer ersten Analyse der ersten angereicherten Probe und einer zweiten Analyse der zweiten angereicherten Probe;
wobei die erste Zielanreicherung und die zweite Zielanreicherung jeweils umfassen:
Hybridisieren einer ersten zielspezifischen Region einer ersten Brückensonde an eine erste Zielsequenz eines Moleküls mit einer Sequenz, die der Genomregion entspricht, wobei eine erste Adaptor-Andocksequenz der ersten Brückensonde an eine erste Brückenbindungssequenz einer Adaptor-Ankersonde gebunden ist; und
Hybridisieren einer zweiten zielspezifischen Region einer zweiten Brückensonde an eine zweite Zielsequenz des Moleküls mit einer Sequenz, die der Genomregion entspricht, wobei eine zweite Adaptor-Andocksequenz der zweiten Brückensonde an eine zweite Brückenbindungssequenz der Adaptor-Ankersonde gebunden ist.

8. Verfahren nach Anspruch 7, bei dem
(i) die erste Analyse eine Sequenzanalyse und die zweite Analyse eine Methylierungsanalyse ist;
(ii) die erste Analyse eine erste Sequenzanalyse und die zweite Analyse eine zweite Sequenzanalyse ist, wobei die erste Sequenzanalyse mit einer anderen Sequenzierungstiefe durchgeführt wird als die zweite Sequenzanalyse;
(iii) die Adaptor-Ankersonde eine Bindungseinheit umfasst und das Verfahren zudem das Anbringen der Bindungseinheit an einen Träger und das Abtrennen des Trägers mit der angebrachten Bindungseinheit von den ungebundenen Nukleinsäuren umfasst;
(iv) das erste oder zweite Panel aus einer oder mehreren Genomregionen ein Panel von Genomregionen umfasst, die mit differentiell methylierten Regionen, mit Hypermethylierung bei Krebs, mit epigenetischen Modifikationen, mit Introns, mit Promotoren oder mit anderen regulatorischen Sequenzen assoziiert sind,
wobei vorzugsweise das erste Panel aus einer oder mehreren Genomregionen ein Panel von Genomregionen umfasst, die mit Mutations-Hotspots assoziiert sind, und das zweite Panel aus einer oder mehreren Genomregionen ein Panel von Genomregionen umfasst, die mit methylierten Regionen assoziiert sind;
(v) das erste oder zweite Panel aus einer oder mehreren Genomregionen ein Panel von Genomregionen umfasst, die mit Mutations-Hotspots, Onkogenen, Tumorsuppressorgenen, Onkogen-Exons, Tumorsuppressor-Exons oder regulatorischen Regionen assoziiert sind;
(vi) die erste angereicherte Probe mittels Sequenzierung analysiert wird, um Mutationen zu bestimmen, während die zweite angereicherte Probe vor der Sequenzierung einer Bisulfitbehandlung oder einer enzymatischen Behandlung unterzogen wird, um die Methylierung zu bestimmen;
(vii) die Analyse der ersten angereicherten Probe vor der Durchführung eines zweiten Zielanreicherungsschritts erfolgt,
wobei vorzugsweise die Analyse der ersten angereicherten Probe dazu verwendet wird, ein zweites Panel für den zweiten Anreicherungsschritt auszuwählen;
und/oder
(viii) das Verfahren zudem die Durchführung einer dritten, vierten oder weiteren Runde der Zielanreicherung mit einem dritten, vierten oder weiteren Panel von Genomregionen umfasst.

9. erfahren nach Anspruch 8, wobei
(i) das Verfahren zudem das Anbringen von Adaptern an die 5'-Enden oder die 3'-Enden von Nukleinsäuremolekülen der Vielzahl von Nukleinsäuremolekülen umfasst; und/oder
(ii) die Nukleinsäuremoleküle DNA, genomische DNA, mitochondriale DNA, virale DNA, cDNA, zellfreie DNA, synthetische DNA, mRNA, tRNA, microRNA, snRNA, piRNA, kleine nicht-kodierende RNA, polysomale RNA, Intron-RNA, prä-mRNA, virale RNA oder zellfreie RNA sind.

10. Nukleinsäurekomplex, umfassend ein Template-Nukleinsäuremolekül,
wobei ein 5'-Ende oder ein 3'-Ende des Template-Nukleinsäuremoleküls an einem Adapter angebracht ist;
wobei eine erste Zielsequenz des Template-Nukleinsäuremoleküls an eine erste zielspezifische Region einer ersten Brückensonde hybridisiert ist und eine zweite Zielsequenz des Template-Nukleinsäuremoleküls an eine zweite zielspezifische Region einer zweiten Brückensonde hybridisiert ist; und
eine erste Adaptor-Andocksequenz der ersten Brückensonde an eine erste Brückenbindungssequenz einer Adaptor-Ankersonde gebunden ist und eine zweite Adaptor-Andocksequenz der zweiten Brückensonde an eine zweite Brückenbindungssequenz der Adaptor-Ankersonde gebunden ist.

11. Nukleinsäurekomplex nach Anspruch 10 zur Verwendung in einem Verfahren zur Diagnose oder zum Erfassen einer Krankheit oder eines Zustands.

12. Nukleinsäurekomplex zur Verwendung nach Anspruch 11, wobei die Krankheit oder der Zustand mit Methylierungsanomalien in Zusammenhang steht,
wobei vorzugsweise das Verfahren die Erzeugung des Methylierungsmusters von zellfreier DNA als Marker für Krebs umfasst.

13. Nukleinsäurekomplex zur Verwendung nach Anspruch 11 oder 12, wobei die Krankheit oder der Zustand ausgewählt ist aus:
(i) einer Krebserkrankung,
wobei die Krebserkrankung vorzugsweise aus einer Gruppe ausgewählt ist, bestehend aus Dickdarmkrebs, Brustkrebs, Leberkrebs, Blasenkrebs, Wilms-Tumor, Eierstockkrebs, Speiseröhrenkrebs, Prostatakrebs, Knochenkrebs, hepatozellulärem Karzinom, Glioblastom, Plattenepithelkarzinom der Lunge, Schilddrüsenkarzinom oder Leukämie;
(ii) einer neurologischen Erkrankung, ausgewählt aus einer Gruppe bestehend aus Alzheimer-Krankheit, Autismus-Spektrum-Störung, Rett-Syndrom und Schizophrenie;
(iii) einer Immundefizienz;
(iv) einer Hauterkrankung;
(v) einer Autoimmunerkrankung, ausgewählt aus der Gruppe bestehend aus okulärem Behçet-Syndrom, systemischem Lupus erythematodes, rheumatoider Arthritis und Multipler Sklerose;
(vi) einer Infektion;
(vii) einer Stoffwechselerkrankung; oder
(viii) einer Erkrankung, ausgewählt aus dem Beckwith-Wiedemann-Syndrom, dem Prader-Willi-Syndrom oder dem Angelman-Syndrom.

14. Nukleinsäurekomplex zur Verwendung nach einem der Ansprüche 11 bis 13, wobei
(i) das Verfahren eine kombinierte gezielte Methylierungssequenzierung und Mutationsanalyse anhand zellfreier DNA zur Krebsfrüherkennung und -behandlung umfasst;
(ii) das Verfahren das Nachweisen gewebespezifischer Methylierungsveränderungen in Krebsgenomen zum Erfassen zirkulierender Tumor-DNA im Plasma von Patienten mit Krebs im Frühstadium oder mit Rezidiv umfasst;
(iii) das Verfahren das Nachweisen des Ursprungs eines Tumors mittels gewebespezifischer miRNA- und DNA-Methylierungsmarker umfasst;
(iv) das Verfahren das Bestimmen von Methylierungsgraden oder fraktionellen Beiträgen verschiedener Gewebe zu einem zellfreien DNA-Gemisch umfasst;
und/oder
(v) das Verfahren das Nachweisen von fetaler DNA oder fetalen Anomalien unter Verwendung einer mütterlichen Probe umfasst.

## Revendications

1. Procédé comprenant :
l'obtention d'une molécule d'acide nucléique matrice attachée à un adaptateur en 5' ou à un adaptateur en 3' ;
l'hybridation d'une première région spécifique cible d'une première sonde de pontage à une première séquence cible de la molécule d'acide nucléique matrice, dans lequel une première séquence d'amarrage d'adaptateur de la première sonde de pontage est configurée pour s'hybrider à une première séquence de liaison de pont d'une sonde d'ancrage d'adaptateur ; et
l'hybridation d'une seconde région spécifique cible d'une seconde sonde de pontage à une seconde séquence cible de la molécule d'acide nucléique matrice, dans lequel une seconde séquence d'amarrage d'adaptateur de la seconde sonde de pontage est configurée pour s'hybrider à une seconde séquence de liaison de pont de la sonde d'ancrage d'adaptateur,
dans lequel la première sonde de pontage et la seconde sonde de pontage comprennent chacune un ADN ou un ARN.

2. Procédé selon la revendication 1,
(i) dans lequel la première sonde de pontage et/ou la seconde sonde de pontage comprennent de l'uracile ;
(ii) dans lequel la première sonde de pontage et/ou la seconde sonde de pontage comprennent de la cytosine méthylée ;
(iii) dans lequel la première sonde de pontage et/ou la seconde sonde de pontage comprennent un ou plusieurs codes-barres moléculaires ;
(iv) dans lequel la première sonde de pontage et/ou la seconde sonde de pontage comprennent un ou plusieurs fragments de liaison ;
(v) dans lequel la première sonde de pontage et/ou la seconde sonde de pontage comprend une étiquette, de préférence dans lequel l'étiquette est une étiquette fluorescente, une étiquette radioactive ou de la biotine ;
et/ou
(vi) dans lequel la première sonde de pontage et/ou la seconde sonde de pontage comprennent un lieur qui comprend un polymère non acide nucléique.

3. Procédé selon la revendication 1 ou 2,
(i) dans lequel la molécule d'acide nucléique matrice est de l'ADN génomique, de l'ADN mitochondrial, de l'ADN viral, de l'ADNc ou de l'ADN synthétique ;
(ii) dans lequel la molécule d'acide nucléique matrice est de l'ADN double-brin, de l'ADN simple-brin, de l'ADN fragmenté ou de l'ADN endommagé ;
(iii) dans lequel la molécule d'acide nucléique matrice est de l'ADN acellulaire, facultativement :
(a) dans lequel l'ADN acellulaire provient d'une source fœtale ;
ou
(b) dans lequel l'ADN acellulaire est de l'ADN tumoral circulant ;
(iv) dans lequel la molécule d'acide nucléique matrice est de l'ARNm, de l'ARNt, du microARN, de l'ARNsn, de l'ARNpi, un petit ARN non codant, de l'ARN polysomal, de l'ARN intronique, du pré-ARNm, de l'ARN viral ou de l'ARN acellulaire ;
(v) dans lequel la molécule d'acide nucléique matrice est un acide nucléique modifié comprenant une ou plusieurs bases hétérocycliques,
facultativement dans lequel les une ou plusieurs bases hétérocycliques sont choisies dans un groupe constitué de purines ou pyrimidines méthylées, purines ou pyrimidines acylées, et riboses alkylés ;
(vi) dans lequel la molécule d'acide nucléique matrice est un acide nucléique modifié comprenant un ou plusieurs fragments sucre modifiés,
facultativement dans lequel les un ou plusieurs fragments sucre modifiés sont choisis dans un groupe constitué de l'acide nucléique à hexitol, l'acide nucléique peptidique, l'acide nucléique verrouillé, l'acide nucléique à thréose et l'acide nucléique à glycérol ; et/ou
(vii) dans lequel l'acide nucléique matrice est un acide nucléique endommagé, facultativement :
(a) dans lequel l'acide nucléique endommagé comprend des bases altérées ou manquantes et/ou une ossature modifiée ;
et/ou
(b) dans lequel l'acide nucléique endommagé a été endommagé par oxydation, radiation, mutation aléatoire ou traitement au bisulfite.

4. Procédé selon l'une quelconque des revendications 1 à 3, comprenant en outre le traitement de la molécule d'acide nucléique matrice par un réactif d'essai de méthylation après l'hybridation de la première région spécifique cible et l'hybridation de la seconde région spécifique cible,
de préférence dans lequel le réactif d'essai de méthylation est du bisulfite ou une enzyme qui modifie la cytosine méthylée.

5. Procédé selon la revendication 4, comprenant en outre le séquençage des molécules d'acide nucléique matrices et l'analyse des données de séquençage par méthylation selon un procédé comprenant les étapes suivantes consistant à :
compter le nombre total (N) de sites CpG de chaque molécule séquencée ;
compter le nombre (M) de sites CpG méthylés de chaque molécule séquencée ;
calculer, pour chaque molécule séquencée, la fraction (f) de sites CpG méthylés en divisant le nombre de sites CpG méthylés (M) par le nombre total de sites CpG (N) ; et
appliquer un modèle de classification descendante, dans lequel chaque paire de données (N, f) représente un point de données dans le modèle de classification descendante.

6. Procédé selon la revendication 5,
(i) comprenant en outre l'application d'un seuil filtre d'hyper-méthylation (f0), dans lequel chaque molécule séquencée avec une fraction de méthylation supérieure au seuil filtre d'hyper-méthylation (f0) est comptée comme étant une molécule hyper-méthylée,
facultativement :
(a) dans lequel le seuil filtre d'hyper-méthylation (f0) est fixé sur la base de l'analyse de méthylation dans un tissu normal ou un échantillon provenant d'un sujet sain ;
(b) dans lequel le seuil filtre d'hyper-méthylation (f0) est fixé à 0,2, 0,3, 0,4, 0,5, 0,6, 0,7, 0,8 ou 0,9 ;
ou
(c) dans lequel le seuil filtre d'hyper-méthylation (f0) est fixé à 0,5, 1, 1,5, 2, 2,5 ou 3 écarts-types par rapport à la fraction de méthylation moyenne dans un échantillon de tissu normal ou un échantillon provenant d'un sujet sain ;
et/ou
(ii) comprenant en outre l'application d'un seuil supplémentaire N0 afin de ne compter que des lectures avec N > N0.

7. Procédé d'enrichissement cible basé sur une hybridation séquentielle comprenant :
l'obtention d'un échantillon comprenant une pluralité de molécules d'acide nucléique ; la réalisation d'un premier enrichissement cible afin d'enrichir en molécules d'acide nucléique comprenant des séquences correspondant à un premier panel d'une ou plusieurs régions du génome, générant ainsi un premier échantillon enrichi comprenant des acides nucléiques enrichis en séquences correspondant au premier panel d'une ou plusieurs régions du génome et un échantillon restant comprenant des acides nucléiques appauvris en séquences correspondant au premier panel d'une ou plusieurs régions du génome ;
la réalisation d'un second enrichissement cible sur l'échantillon restant afin d'enrichir en molécules d'acide nucléique comprenant des séquences correspondant à un deuxième panel d'une ou plusieurs régions du génome, générant ainsi un second échantillon enrichi comprenant des acides nucléiques enrichis en séquences correspondant au deuxième panel d'une ou plusieurs régions du génome ; dans lequel le premier panel d'une ou plusieurs régions du génome et le deuxième panel d'une ou plusieurs régions du génome sont différents ; et
la réalisation d'une première analyse du premier échantillon enrichi et d'une seconde analyse du second échantillon enrichi ;
dans lequel le premier enrichissement cible et le second enrichissement cible comprennent chacun :
l'hybridation d'une première région spécifique cible d'une première sonde de pontage à une première séquence cible d'une molécule ayant une séquence correspondant à la région du génome, dans lequel une première séquence d'amarrage d'adaptateur de la première sonde de pontage est liée à une première séquence de liaison de pont d'une sonde d'ancrage d'adaptateur ; et
l'hybridation d'une seconde région spécifique cible d'une seconde sonde de pontage à une seconde séquence cible de la molécule ayant une séquence correspondant à la région du génome, dans lequel une seconde séquence d'amarrage d'adaptateur de la seconde sonde de pontage est liée à une seconde séquence de liaison de pont de la sonde d'ancrage d'adaptateur,

8. Procédé selon la revendication 7,
(i) dans lequel la première analyse est une analyse de séquence et la seconde analyse est une analyse de méthylation ;
(ii) dans lequel la première analyse est une première analyse de séquence, et la seconde analyse est une seconde analyse de séquence, dans lequel la première analyse de séquence est réalisée à une profondeur de séquençage différente de la seconde analyse de séquence ;
(iii) dans lequel la sonde d'ancrage d'adaptateur comprend un fragment de liaison et le procédé comprend en outre l'attache du fragment de liaison à un support et la séparation du support ayant le fragment de liaison attaché des acides nucléiques non liés ;
(iv) dans lequel le premier ou le deuxième panel d'une ou plusieurs régions du génome comprend un panel de régions du génome associées à des régions différentiellement méthylées, à une hyper-méthylation dans un cancer, à des modifications épigénétiques, à des introns, à des promoteurs ou à d'autres séquences régulatrices, de préférence dans lequel le premier panel d'une ou plusieurs régions du génome comprend un panel de régions du génome associées à des points chauds de mutation et dans lequel le deuxième panel d'une ou plusieurs régions du génome comprend un panel de régions du génome associées à des régions méthylées ;
(v) dans lequel le premier ou le deuxième panel d'une ou plusieurs régions du génome comprend un panel de régions du génome associées à des points chauds de mutation, des oncogènes, des gènes suppresseurs de tumeur, des exons d'oncogène, des exons de suppresseur de tumeur ou des régions régulatrices ;
(vi) dans lequel le premier échantillon enrichi est analysé par séquençage pour évaluer des mutations tandis que le second échantillon enrichi est traité au bisulfite ou traité enzymatiquement avant séquençage pour évaluer la méthylation ;
(vii) dans lequel l'analyse du premier échantillon enrichi est réalisée avant la réalisation d'une seconde étape d'enrichissement cible,
de préférence dans lequel l'analyse du premier échantillon enrichi est utilisée pour sélectionner un deuxième panel pour la seconde étape d'enrichissement ;
et/ou
(viii) dans lequel le procédé comprend en outre la réalisation de troisième, quatrième ou plus cycles d'enrichissement cible avec des troisième, quatrième ou plus panels de régions du génome.

9. Procédé selon la revendication 8,
(i) dans lequel le procédé comprend en outre l'attache d'adaptateurs aux extrémités 5' ou aux extrémités 3' des molécules d'acide nucléique de la pluralité de molécules d'acide nucléique ;
et/ou
(ii) dans lequel les molécules d'acide nucléique sont de l'ADN, de l'ADN génomique, de l'ADN mitochondrial, de l'ADN viral, de l'ADNc, de l'ADN acellulaire, de l'ADN synthétique, de l'ARNm, de l'ARNt, un microARN, de l'ARNsn, de l'ARNpi, un petit ARN non codant, de l'ARN polysomal, de l'ARN intronique, du pré-ARNm, de l'ARN viral ou de l'ARN acellulaire.

10. Complexe d'acide nucléique comprenant une molécule d'acide nucléique matrice, dans lequel une extrémité 5' ou une extrémité 3' de la molécule d'acide nucléique matrice est attachée à un adaptateur ;
dans lequel une première séquence cible de la molécule d'acide nucléique matrice est hybridée à une première région spécifique cible d'une première sonde de pontage et une seconde séquence cible de la molécule d'acide nucléique matrice est hybridée à une seconde région spécifique cible d'une seconde sonde de pontage ; et
dans lequel une première séquence d'amarrage d'adaptateur de la première sonde de pontage est liée à une première séquence de liaison de pont d'une sonde d'ancrage d'adaptateur et une seconde séquence d'amarrage d'adaptateur de la seconde sonde de pontage est liée à une seconde séquence de liaison de pont de la sonde d'ancrage d'adaptateur.

11. Complexe d'acide nucléique selon la revendication 10 pour utilisation dans un procédé de diagnostic ou de détection d'une maladie ou d'une affection.

12. Complexe d'acide nucléique pour utilisation selon la revendication 11, dans lequel la maladie ou l'affection est associée à des anomalies de méthylation,
de préférence dans lequel le procédé comprend la génération du profil de méthylation d'ADN acellulaire comme marqueur de cancer.

13. Complexe d'acide nucléique pour utilisation selon la revendication 11 ou 12, dans lequel la maladie ou l'affection est choisie parmi :
(i) un cancer,
de préférence dans lequel le cancer est choisi dans un groupe constitué du cancer du côlon, du cancer du sein, du cancer du foie, du cancer de la vessie, du cancer de Wilms, du cancer de l'ovaire, du cancer de l'œsophage, du cancer de la prostate, du cancer des os, du carcinome hépatocellulaire, du glioblastome, du cancer du poumon à cellules squameuses, du carcinome thyroïdien ou de la leucémie ;
(ii) une maladie neurologique choisie dans un groupe constitué de la maladie d'Alzheimer, du trouble du spectre de l'autisme, du syndrome de Rett et de la schizophrénie ;
(iii) une immunodéficience ;
(iv) une maladie de la peau ;
(v) une maladie auto-immune choisie dans le groupe constitué de la maladie de Behçet oculaire, du lupus érythémateux systémique, de la polyarthrite rhumatoïde et de la sclérose en plaques ;
(vi) une infection ;
(vii) une maladie métabolique ; ou
(viii) une affection choisie parmi le syndrome de Beckwith-Wiedemann, le syndrome de Prader-Willi ou le syndrome d'Angelman.

14. Complexe d'acide nucléique pour utilisation selon l'une quelconque des revendications 11 à 13,
(i) dans lequel le procédé comprend un séquençage par méthylation ciblé et une analyse de mutations combinés à partir d'ADN acellulaire pour une détection et une prise en charge précoces de cancer ;
(ii) dans lequel le procédé comprend la détection de variations de méthylation spécifiques à un tissu dans des génomes de cancer pour détecter un ADN tumoral circulant dans le plasma de patients atteints d'un cancer au stade précoce ou d'un cancer récidivant ;
(iii) dans lequel le procédé comprend la détection de l'origine d'une tumeur avec des marqueurs de méthylation d'ARNmi et ADN spécifiques à un tissu ;
(iv) dans lequel le procédé comprend la détermination de niveaux de méthylation ou de contributions fractionnaires de différents tissus à un mélange d'ADN acellulaire ;
et/ou
(v) dans lequel le procédé comprend la détection d'ADN fœtal ou d'anomalies fœtales au moyen d'un échantillon maternel.
